# EUROPEAN PATENT SPECIFICATION

(11) **EP 2 049 469 B1**
(45) Date of publication and mention of the grant of the patent: **23.02.2011**
(21) Application number: 07787875.9
(22) Date of filing: 25.07.2007
(51) Int. Cl.: C07C 257/22, C07C 251/18, C07C 233/58

(54) **PROCESS FOR PREPARING AMIDRAZONES**
VERFAHREN ZUR HERSTELLUNG VON AMIDRAZONEN
PROCÉDÉ DE FABRICATION D'AMIDRAZONES

(30) Priority: 03.08.2006 EP 06118365
(43) Date of publication of application: 22.04.2009
(73) Proprietor: BASF SE, 67056 Ludwigshafen (DE)
(72) Inventor: ZIERKE, Thomas, 67459 Böhl-iggelheim (DE); KORADIN, Christopher, 67067 Ludwigshafen (DE)
(86) International application number: PCT/EP2007/057645
(87) International publication number: WO 2008/015133

(56) References cited:
- EP-A1- 0 604 798
- WO-A-2005/053401
- DE-A1- 2 944 849
- J. BEGER ETAL: "Phenylamino-2-imidazoline und 1,2,5,6-Tetrahydro-1,2,4-triazine aus N-(2-Chloralkyl)-imidchloriden" MONATSHEFTE FUR CHEMIE., vol. 112, no. 8-9, 1981, pages 959-971, XP002423216 ATSPRINGER VERLAG. WIEN. cited in the application
- NISHII YOSHINORI ET AL: "Chirality exchange from sp3 central chirality to axial chirality: benzannulation of optically active diaryl-2,2-dichlororcyclopropylmethanols to axially chiral alpha-arylnaphthalenes" JOURNAL OF THE AMERICAN CHEMICAL SOCIETY., vol. 126, no. 17, 2004, pages 5358-5359, XP002463845 USAMERICAN CHEMICAL SOCIETY, WASHINGTON, DC.
- DATABASE CHEMCATS [Online] 18 May 2007 (2007-05-18), XP002463846 Database accession no. 2023005489
- DATABASE REGISTRY [Online] 15 May 2002 (2002-05-15), XP002463847
- DATABASE CHEMCATS [Online] 18 May 2007 (2007-05-18), XP002463848 Database accession no. 2023708137

## Description

The present invention relates to a process for preparing amidrazones of formula I 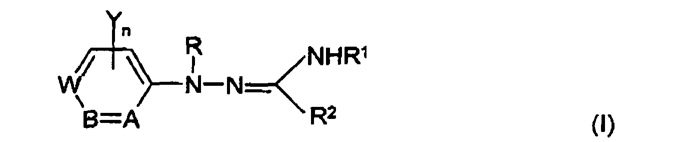 wherein
- R: is hydrogen;
- R¹: is C₁-C₁₀-alkyl;
- R²: is C₃-C₁₀-cycloalkyl which is unsubstituted or substituted with 1 to 5 halogen atoms and/or 1 to 3 C₁-C₆-alkyl groups and/or 1 to 3 C₁-C₈-haloalkyl groups;
- A: is C-R³ or N;
- B: is C-R⁴ or N;
- W: is C-R⁵ or N;
with the proviso that one of A, B and W is other than N;
- R³, R⁴, R⁵: are each independently hydrogen, halogen, nitro, cyano, amino, mercapto, hydroxy, C₁-C₁₀-alkyl, C₂-C₁₀-alkenyl, C₂-C₁₀-alkynyl, C₃-C₆-cycloalkyl, C₁-C₆-alkoxy, C₁-C₆-alkylamino, di(C₁-C₆-alkyl)-amino, C₁-C₆-alkylthio, C₁-C₆-alklsulfonyl, C₁-C₆-alkylsulfonyl or a 5- to 6-membered aromatic ring which may contain 1 to 4 heteroatoms selected from oxygen, sulfur and nitrogen;
- Y: is hydrogen, halogen, cyano, nitro, amino, hydroxy, mercapto, C₁-C₆-alkyl, C₂C₁₀-alkenyl, C₂-C₁₀-alkynyl, C₃-C₆-cycloalkyl, C₁-C₆-alkoxy, C₁-C₆-alkylamino, di(C₁-C₆)-alkylamino, C₁-C₆-alkylthio, C₁-C₆-alkylsulfonyl, or C₁-C₆-alkylsulfinyl; and
- n: is 0, 1, or 2
or a salt thereof.

Amidrazones are useful pest control agents, e.g. as described in EP-A 604 798, and also in J. A Furch et al., "Amidrazones: A New Class of Coleopteran Insecticides", ACS Symposium Series 686, Am. Chem. Soc., 1998, Chapter 18, pp. 178; and D. G. Kuhn et al., "Cycloalkyl-substituted Amidrazones: A Novel Class of Insect Control Agents", ACS Symposium Series 686, Am. Chem. Soc., 1998, Chapter 19, pp. 185.

Each of these references describes the preparation of a broad range of amidrazones by the route as shown in the following scheme:

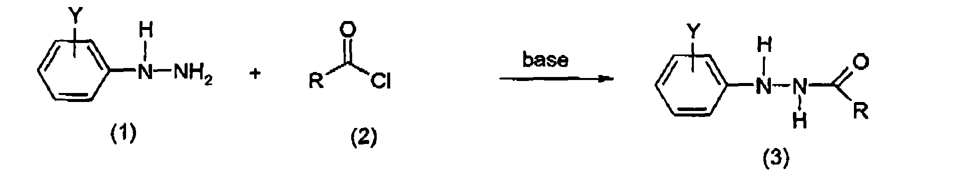

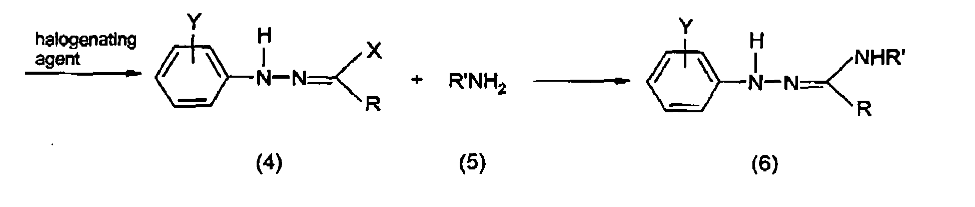

This synthesis route involves reacting a substituted arylhydrazine (1) with an appropriate acid chloride (2) in the presence of a base to obtain the arylhydrazide (3). The arylhydrazide (3) is then reacted with a halogenating agent such as, for example, thionylchloride to give the arylhydrazinoyl halide (4). The reaction of the arylhydrazinoyl halide (4) with the amine (5) yields the desired amidrazone (6).

The known route starts from the relatively expensive arylhydrazine (1), which is undesirable due to the corresponding losses in yield during the subsequent reaction steps. Another problem of the known preparation process is the isolation of the amidrazone (6) in pure or relatively pure form. This can only be achieved through laborious purification of the precursor arylhydrazinoyl halide (4) such as, for example, by silica filtration. In addition, a further purification of the final product through its hydrochloride salt may be required, which causes additional costs.

It is also known in the art that only a few specific amidrazone derivatives can be prepared by reacting aryl hydrazines with imide derivatives.

J. Beger et al., Monatshefte fuer Chemie, Vol. 112, No. 8-9, 1981, pp. 959-971, discloses the synthesis of certain 1-phenylamino-2-imidazolines and 1,2,5,6-tetrahydro-1,2,4-triazines from N-(2-chloroalkyl)-imide chlorides and phenylhydrazines via amidrazones hydrochlorides as intermediates. The provided amidrazone hydrochlorides show a great tendency to undergo spontaneous cyclization to the heterocyclic compounds. Further, the preparation of the amidrazone hydrochlorides disclosed therein requires excess amounts of the expensive phenylhydrazine as starting material which is undesirable from the point of view of costs. Despite the use of such excess amounts, the described process leads to low yields relative to the phenylhydrazine.

P. Nuhn et al., Pharmazie, Vol. 48, No. 5, 1993, pp. 340-342, discloses the reaction of certain open-chain aryl-substituted amidrazones by reaction of N-aryl benzimide chlorides with arylhydrazines. However, the isolation of the amidrazones by repeated crystallization is difficult due to the presence of undesired by-products, resulting in low yield and purity of the final product.

The compound 2,2-dibromo-1-methyl-N-(1-phenylethyl)-cyclopropanecarboxamide has been published in the database CHEMCATS (Database accession No. 2023005489).

DE 2944849 A1 discloses a cyclopropyl-substituted chloroimine derivative having a dimethyl group and a dichloroethenyl group as substituents on the ring (see page 52, lines 10-15).

Nishii Yoshinori et al "Chirality exchange from sp3 central chirality to axial chirality: benzannulation of optically active diaryl-2,2-dichlorocyclopropylmethanols to axially chiral α-arylnaphthalenes", J. Am. Chem. Soc., Vol. 126, No. 17, 2004, pages 5358-5359 discloses the compound N-(1S)-1-phenylethyldichlorocyclopropylcarboxamide (see page 5359, reference 8).

The compound 2,2-dibromo-1-methyl-N-(1-methylpropyl)-cyclopropanecarboxamide has been published in the database REGISTRY (Database registration No. 415944-13-7).

Thus, there remains a need to provide an economical and industrially feasible process for the preparation of the specific amidrazones of the formula I or a salt thereof which affords the compounds I or a salt thereof in high yields and high purity.

Surprisingly, it has now been found that the specific amidrazones of formula I or a salt thereof are obtained in high yields and high purity when an arylhydrazine of formula II 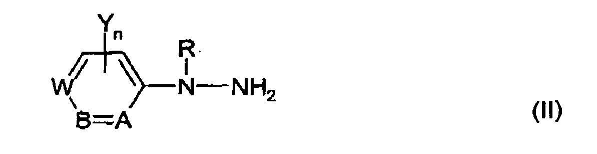 wherein R, A, B, W, Y and n are each as defined above, is reacted with an imidoyl compound of formula III 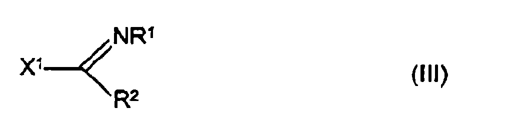 wherein R¹ and R² are each as defined above and X¹ is halogen, C₁-C₆-alkoxy, C₁-C₆-alkylsulfonate, C₁-C₆-haloalkylsulfonate, C₆-C₁₂arylsulfonate, C₁-C₆-alkylthio, C₁-C₆-alkylphosphate, or C₆-C₁₂-arylphosphate, optionally in the presence of a base.

The present invention therefore provides a process for preparing the amidrazones of formula I or a salt thereof, comprising reacting an arylhydrazine of formula II with an imidoyl compound of formula III, optionally in the presence of a base.

Another advantage of the inventive process is that relatively cheap starting materials can be used for the production of the imidoyl compound of formula III as the second reactant

The imidoyl compound of formula III can be obtained in a similar manner to the known prior art methods from an amide of formula IV 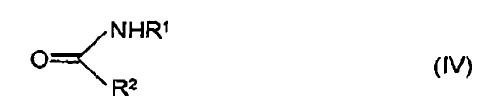 wherein R¹ and R² are each as defined above, by reacting the amide IV with an agent capable of converting the amide IV into the imidoyl compound III. Accordingly, the process according to the invention preferably also comprises the preparation of the imidoyl compound of formula III by this route.

The imidoyl compounds of the formula III are novel wherein
- R¹: is C₁-C₁₀-alkyl, C₃-C₁₃-alkenyl, C₃-C₁₀-alkynyl, C₃-C₁₂-cycloalkyl, di(C₁-C₆-alkyl)- amino, C₁-C₆-alkylsulfonyl or C₁-C₆-alkylsulfinyl, wherein in each of the above radicals the alkyl, alkenyl, alkynyl or cycloalkyl groups are unsubstituted or substituted with 1 to 3 substituents independently selected from halogen, nitro, cyano, C₁-C₆-alkoxy, C₁-C₆-haloalkoxy, C₁-C₆-alkylthio, C₁-C₆-haloalkylthio, C₁-C₆-alkylsulfonyl, C₁-C₆-alkylsulfinyl, C₁-C₆-haloalkylsulfonyl, C₁-C₆-haloalkylsulfinyl, C₃-C₆-cycloalkyl, phenyl and pyridyl:
- R²: is cyclopropyl which is substituted with 1 to 3 halogen atoms and/or 1 to 3 C₁-C₈- alkyl groups and/or 1 to 3 C₁-C₈-haloalkyl groups; and
- X¹: is halogen, C₁-C₆-alkoxy, C₁-C₆-alkylsulfonate, C₁-C₆-haloalkylsulfonate, C₁-C₁₂-arylsulfonate, C₁-C₆alkylthio, C₁-C₆-alkylphosphate, or C₆-C₁₂-arylphosphate.

Therefore, these compounds likewise form part of the subject matter of the present invention as starting materials or intermediates in the process according to the invention.

Further, the amide of the formula IV used in the process for the preparation of the imidoyl compounds III may in turn be obtained in a similar manner to the known prior art processes from a compound of formula V 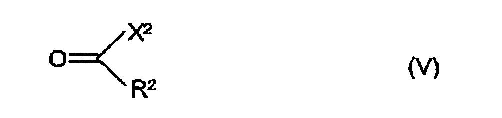 wherein X² is a suitable leaving group and R² is as defined above, by reacting the compound V with an amine of formula VI

R¹-NH₂ (VI)

wherein R¹ is as defined above. Accordingly, the process according to the invention preferably also comprises the preparation of the amides of the formula IV by this route.

The amides of the formula IV are novel wherein
- R¹: is C₁-C₁₀alkyl, C₃-C₁₀-alkenyl, C₃-C₁₀-alkynyl, C₃-C₁₂-cycloalkyl, di(C₁-C₆-alkyl)- amino, C₁-C₆-alkylsulfonyl or C₁-C₆-alkylsulfinyl, wherein in each of the above radicals the alkyl, alkenyl, alkynyl or cycloalkyl groups are unsubstituted or substituted with 1 to 3 substituents independently selected from halogen, nitro, cyano, C₁-C₆-alkoxy, C₁-C₆-haloalkoxy, C₁-C₆-alkylthio, C₁-C₆-haloalkylthio, C₁-C₆-alkylsulfonyl, C₁-C₆-alkylsulfinyl, C₁-C₆-haloalkylsulfonyl, C₁-C₆-haloalkylsulfinyl and C₃-C₆-cycloalkyl;
- R²: is a cyclopropyl group of formula VII 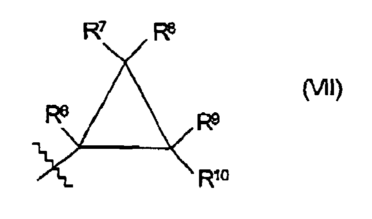
wherein R⁸ is C₁-C₈-alkyl, R⁷ and R⁸ are both chlorine, and R⁹ and R¹⁰ are both hydrogen.

Accordingly, these compounds are also contemplated by the present invention as starting materials or intermediates in the process according to the invention.

In the definitions of the variables specified in the above formulae I to VII, collective terms are used which are generally representative of particular substituents. The term Cₐ-C_{b} specifies the number of carbon atoms in the particular substituents or substituent moiety which is possible in each case. Other definitions are as follows:
"Halogen" will be taken to mean fluorine, chlorine, bromine, and iodine.
The term "halo" refers to fluoro, chloro, bromo, and iodo.

The term "alkyl" as used herein refers to a branched or unbranched saturated hydrocarbon group having 1 to 10 carbon atoms, especially C₁-C₆-alkyl such as methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl.

The term "haloalkyl" as used herein refers to a straight-chain or branched alkyl groups having 1 to 10 carbon atoms (as mentioned above), where some or all of the hydrogen atoms in these groups may be replaced by halogen atoms as mentioned above, for example C₁-C₂-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl.

Similarly, "alkoxy" and "alkylthio" refer to straight-chain or branched alkyl groups having 1 to 6 carbon atoms (as mentioned above) bonded through oxygen or sulfur linkages, respectively, at any bond in the alkyl group. Examples include methoxy, ethoxy, propoxy, isopropoxy, methylthio, ethylthio, propylthio, isopropylthio, and n-butylthio.

Similarly, "alkylsulfinyl", "alkylsulfonyl" and "alkylsulfonate" refer to straight-chain or branched alkyl groups having 1 to 6 carbon atoms (as mentioned above) bonded through -S(=O)-, -S(=O)₂- or -S(=O)₂-O- linkages, respectively, at any bond in the alkyl group. Examples include methylsulfinyl, methylsulfonyl and methylsulfonate.

Similarly, "alkylamino" refers to a nitrogen atom which carries 1 or 2 straight-chain or branched alkyl groups having 1 to 6 carbon atoms (as mentioned above) which may be the same or different. Examples include methylamino, dimethylamino, ethylamino, diethylamino, methylethylamino, isopropylamino, or methylisopropylamino.

The term "alkylcarbonyl" refers to straight-chain or branched alkyl groups having 1 to 6 carbon atoms (as mentioned above) bonded through a -C(=O)- linkage, respectively, at any bond in the alkyl group. Examples include acetyl and propionyl.

The term "alkenyl" as used herein intends a branched or unbranched unsaturated hydrocarbon group having 3 to 10 carbon atoms and a double bond in any position, such as C₃-C₆ alkenyl such as 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl; 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl,2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "alkynyl" as used herein refers to a branched or unbranched unsaturated hydrocarbon group containing at least one triple bond, such as ethynyl, propynyl, 1-butynyl, 2-butynyl and the like.

"Cycloalkyl" refers to a monocyclic 3- to 8-, 10- or 12-membered saturated carbon atom rings, e.g. C₃-C₈-cycloalkyl such as cyclopropyl, cyclobutyl, cyclopentyl, cyclohexyl, cycloheptyl and cyclooctyl.

The term "aryl" refers to an aromatic carbocyclic group having at least one aromatic ring (e.g., phenyl or biphenyl) or multiple condensed rings in which at least one ring is aromatic, (e.g., 1,2,3,4-tetrahydronaphthyl, naphthyl, anthryl, or phenanthryl), each of which may be substituted.

A 5- to 6-membered aromatic ring containing 1 to 4 heteroatoms selected from oxygen, sulfur and nitrogen, intends e.g. 5-membered heteroaryl, containing 1 to 4 nitrogen atoms or 1 to 3 nitrogen atoms and 1 sulfur or oxygen atom, e.g. furyl, thienyl, pyrrolyl, isoxazolyl, isothiazolyl, pyrazolyl, oxazolyl, thiazolyl, imidazolyl, oxadiazolyl, thiadiazolyl, oxadiazolyl, triazolyl, and tetrazolyl; or 6-membered heteroaryl, containing 1 to 4 nitrogen atoms or 1 to 3 nitrogen atoms and 1 sulfur or oxygen atom, e.g. 2-pyridinyl, 3-pyridinyl, 4-pyridinyl, 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl.

A 3- to 6-membered saturated or partially unsaturated ring which contains 1 to 3 heteroatoms selected from oxygen, sulfur and nitrogen intends e.g. a saturated 3- to 6- membered ring containing 1 to 3 heteroatoms selected from nitrogen and oxygen, such as aziridine, pyrrolidine, tetrahydrofuran, tetrahydropyran, or piperidine.

A "suitable leaving group" is any group which can be displaced at the carbon atom by nucleophiles such as nitrogen, oxygen, sulfur, etc. or the anions of these nucleophiles under the reaction conditions of the process of the present invention such as, for example, halogen (e.g. fluorine, chlorine, and bromine), hydroxy, alkoxy, alkylcarboxylate (e.g. acetate), benzoate, alkylsulfonate (e.g. mesylate), haloalkylsulfonate (e.g. trifluoromethylsulfonate), arylsulfonate (e.g. tosylate), alkylthio, alkylphosphate (e.g. diethylphosphate) and arylphosphate. Preferred leaving groups are halogen and C₁-C₆-alkoxy. Examples of halogen are fluorine, chlorine, and bromine, with chlorine being preferred. Preference is also given to C₁-C₄-alkoxy, more preferably methoxy, ethoxy, propoxy, butoxy and isobutoxy, in particular methoxy or ethoxy, with methoxy being most preferred.

The arylhydrazines of formula II are known and can be prepared according to methods described in the art, for example by E. Enders in Houben-Weyl, Vol. X/2 (Stickstoffverbindungen I), Georg Thieme Verlag, Stuttgart, 1967, pp. 169 et sqq. The process as described in DE-A 34 47 211 may also be used for preparing the arylhydrazine II.

To react the arylhydrazine II with the imidoyl compound III, the compounds will be used preferably in a molar ratio of II:III in the range of from 1:1.1 to 1:1.4, in particular from 1:1.3 to 1:1.2 and more preferably from 1;1.1 to 1:1.

The arylhydrazine II is advantageously reacted with the imidoyl compound III at temperatures of at least 20°C, for example in the range from 25°C to 90°C and in particular from 40°C to 80°C. The reaction pressure is of minor importance for the success of the process according to the invention and thus the reaction of arylhydrazine II with the imidoyl compound III can be carried out under reduced pressure, under normal pressure (i.e. atmospheric pressure) or under increased pressure. Preference is given to carrying out the reaction in the region of atmospheric pressure, for example in the range from 0.9 to 1.2 bar. The reaction time can be varied in a wide range and depends on a variety of factors including the heat transfer capacity of the used equipment. The reaction time required for the reaction is generally in the range from 1 to 10 hours, in particular 1 to 5 hours.

The reaction may in principle be carried out in substance. However, preference is given to reacting the arylhydrazine II with the imidoyl compound III in an organic solvent. Suitable solvents are in principle any which are capable of at least partly and preferably fully dissolving the arylhydrazine II and the imidoyl compound III under reaction conditions. Preferred solvents are aprotic. They are in particular those solvents that have a boiling point at atmospheric pressure in the range from 35 °C to 160°C and in particular in the range of 60°C to 130 °C. Preferred solvents are aliphatic and aromatic solvents, in particular aliphatic and aromatic hydrocarbons, halogenated aliphatic and aromatic hydrocarbons, heterocyclic aromatic compounds and mixtures thereof. Particularly preferred solvents are aromatic hydrocarbons, especially alkylbenzenes and even more preferably alkylbenzenes which are mono-, di-, or trialkylsubstituted with each alkyl group containing 1 to 3 carbon atoms, such as, for example, toluene, xylenes (i.e. m-xylene, o-xylene, p-xylene, and mixtures thereof), ethylbenzene, 2-propylbenzene (cumene), 2-isopropyltoluene (o-cymol), 3-isopropyltoluene (m-cymol), 4-isopropyltoluene (p-cymol), 1,3,5-trimethylbenzene (mesitylene) and mixtures thereof, with toluene being the most preferred. Halogenated aromatic hydrocarbons and heterocyclic aromatic compounds are also preferred. Examples of halogenated aromatic hydrocarbons include chlorobenzene, bromobenzene, o-dichlorobenzene, m-dichlorobenzene and mixtures thereof, with preference being given to chlorobenzene. Examples of suitable heterocyclic aromatic compounds include pyridine, 5-ethyl-2-methyl-pyridine, 2-methylpyridine, 3-methylpyridine, 4-methylpyridine and mixtures thereof.

The arylhydrazine II, the imidoyl compound III and optionally the base may be contacted together in any suitable manner. In general, the arylhydrazine II, the imidoyl compound III and optionally the base will be initially charged in a reaction vessel, if appropriate together with the solvent desired, and then the desired reaction conditions will be established. In a preferred embodiment, however, the major amount, especially at least 80 % and more preferably the total amount or virtually the total amount (at least 95%) of the arylhydrazine II may also be charged into a reaction vessel, if appropriate in the desired solvent. The base may then be added thereto and the desired reaction conditions will be established. Following this the major amount, preferably at least 80 % and more preferably the total amount or virtually the total amount (at least 95%) of the imidoyl compound III is added thereto under reaction conditions in the course of the reaction. The imidoyl compound III can be added as such or preferably in the desired solvent

The arylhydrazine II can be reacted with the imidoyl compound III in the presence or in the absence of a base. It is preferred, however, that the reaction of the arylhydrazine II with the imidoyl compound III is carried out in the presence of a base to obtain the amidrazones of formula I. Suitably bases used in this reaction are inorganic compounds, such as, for example, alkali metal hydroxides, alkali hydrogen carbonates, alkali carbonates, alkaline earth metal hydroxides, carbonates or bicarbonates, and alkali hydrides. Advantageously, organic bases, such as tertiary amines, can be used. Preference is given to tertiary amines represented by the formula N(R¹¹(R¹²)(R¹³) wherein R¹¹, R¹² and R¹³ are the same or different and each represents a straight chain or branched chain alkyl or cycloalkyl radical having 1 to 8 carbon atoms, such as methyl, ethyl, propyl, butyl, isopropyl, t-butyl, amyl, cyclohexyl and the like. Examples of suitable tertiary amines are trimethylamine, triethylamine, tripropylamine, triisopropylamine, tri-n-butylamine, tri-sec-butytamine, tri-t-butylamine, tri-n-octylamine, diisopropylethylamine, and dicydohexylethylamine, Particular preference is given to tri-n-butylamine, diisopropyfethylamine and dicyclohexylethylamine. It has been found to be particularly advantageous to use tri-n-butylamine as the base, which has an economically attractive price, is easy to recover from aqueous waste streams and allows to perform the reaction of the arylhydrazine II with the imidoyl compound III in homogeneous solution in a wide range solvents.

Advantageously, the base is employed in a stoichiometric amount or in a slight excess, relative to the arylhydrazine of formula II. Preferably, the base is present in an amount of 1 to 1.5 moles, in particular 1 to 1.3 moles and more preferably 1 to 1.2 moles, relative to 1 mole of the arylhydrazine II.

In the case of conducting the reaction in the absence of a base, a salt of the amidrazone of formula I is obtained, said salt comprising an anionic counterion derived from the moiety X¹ as defined hereinabove, in particular halogenides, alkylsulfonates, haloalkylsulfonates, arylsulfonates, alkylphosphates, or arylphosphates. Preferred salts are halogenides, in particular chlorides.

In case of reacting the arylhydrazine II with the imidoyl compound III in the presence of a base, the resulting amidrazone I (that is, a compound having a basic nitrogen atom at the amine moiety) may subsequently be reacted with a suitable acid to form an acid addition salt of the amidrazone I. "Acid-addition salts" include conventional salts formed from suitable organic or inorganic acids. Exemplary acid-addition salts formed by the compounds of formula I include chlorides, bromides, fluorides, hydrogen sulfates, sulfates, dihydrogen phosphates, hydrogen phosphates, phosphates, nitrates, hydrogen carbonates, carbonates, hexafluorosilicates, hexafluorophosphates, benzoates, and the salts of C₁-C₄-alkanoic acids, preferably formates, acetates, propionates and butyrates. Preferred acid-addition salts are chlorides and hydrogen sulfates.

The amidrazones of formula I can be isolated from the reaction mixture by employing conventional methods, for example by adding water to the reaction mixture, extracting with an organic solvent, concentrating the extract and the like. If the base is employed in an excess relative to arylhydrazine II, an acid (e.g. hydrochloric acid, sulphuric acid and the like) is advantageously added to neutralize the excessive base. In case of preparing a salt of the amidrazones I, isolation may be effected by conventional methods such as filtration.

The process according to the present invention may also lead to isomers of the amidrazones of formula I as defined herein. The term "isomer" is used herein in its broadest sense to include structural isomers (such as, for example, positional isomers and tautomers) and stereoisomers (such as, for example, optical isomers, rotational isomers and E/Z isomers). In cases where the amidrazones of formula I possess asymmetric carbon atoms, the process according to the present invention may result in the racemates and diastereomers as well as the individual enantiometric forms of the amidrazones I as described herein. Mixtures of isomers can be separated into individual isomers according to methods customary for this purpose, e.g. fractional crystallization, adsorption chromatography or other suitable separation processes. Resulting racemates can be separated into antipodes in the usual manner after introduction of suitable salt-forming groupings, e.g. by forming a mixture of diastereosiomeric salts with optically active salt-forming agents, separating the mixture into diastereomeric salts and converting the separated salts into the free compounds. The possible enantiomeric forms may also be separated by fractionation through chiral high performance liquid chromatography columns.

The imidoyl compound of the general formula III used in the process according to the invention can be prepared in a similar manner to the prior art methods for preparing imidoyl compounds by reacting the amide of formula IV with an agent capable of converting the amide IV into the imidoyl compound III, for example in a similar manner to the methods described in Chem. Ber. 93 (1960), pp. 1231-1236 or Liebigs Ann. Chem. 716 (1968), pp. 127-134.

Examples of suitable agents capable of converting the amide IV into the imidoyl compound III are halogenating agents, O-alkylating agents, phosphating agents and sulfonylating agents. Preferred agents for use in the invention are halogenating agents.

For example, if the desired imidoyl compound III is one wherein X¹ is halogen, the amide of formula IV is reacted with an appropriate halogenating agent, preferably a chlorinating or brominating agent Examples of suitable halogenating agents include thionyl chloride, phosgene, oxalyl chloride, sulfuryl chloride, p-toluenesulfonyl chloride, methanesulfonyl chloride, phosphorus pentachloride, phosphorus oxychloride, phosphorus trichloride, thionyl bromide, phosphorus pentabromide, phosphorus tribromide, phosphorus oxybromide and methanesulfonyl bromide. Even more preferred are thionyl chloride, phosgene and oxalyl chloride, with thionyl chloride being the most preferred.

If the desired imidoyl compound III is one wherein X¹ is C₁-C₆-alkoxy, it is possible to react the amide of formula IV with an O-alkylating agent. Suitable O-alkylating agents include, for example, trialkyloxonium salts such as triethyloxonium tetrafluoroborate.

Further, if the desired imidoyl compound III is one wherein X¹ is C₁-C₆-alkylphosphate or C₆-C₁₂-arylphosphate, the amide of formula IV is reacted with an appropriate phosphating agent. As the phosphating agent, phosphoric acid chlorides such as dialkylphosphoric acid chlorides (e.g. diethylphosphoric acid chloride) may be used.

Moreover, if the desired imidoyl compound III is one wherein X¹ is C₁-C₆-alkylsulfonate, C₁-C₆-haloalkylsulfonate or C₆-C₁₂-arylsulfonate, the amide of formula IV is reacted with an appropriate sulfonylating agent. Examples of suitable sulfonylating agents include alkylsulfonic acid anhydrides, haloalkylsulfonic acid anhydrides, arylsulfonic acid anhydrides, alkylsulfonic acid chlorides, haloalkylsulfonic acid chlorides, and arylsulfonic acid chlorides, such as p-toluenesulfonyl chloride or mesitylenesulfonyl chloride.

If the desired imidoyl compound III is one wherein X¹ is C₁-C₆-alkylthio, it can be prepared by converting the amide of formula IV into the corresponding thioamide by treatment with Lawesson's reagent and reacting the thioamide obtained therefrom with an alkylating agent. As the alkylating agent, an alkyl halogenide such as methyl iodide may be used.

In a preferred embodiment of this invention, the amide of formula IV is reacted with a halogenating agent, in particular with any of the halogenating agents specified above and more preferably with a halogenating agent selected from thionyl chloride, phosgene and oxalyl chloride, with thionyl chloride being preferred.

In general, the agent capable of converting the amide IV into the imidoyl compound III will be used in an amount of 1 to 5 moles, in particular 1.2 to 3 moles and more preferably 1.2 to 2 moles, relative to 1 mole of the amide IV.

The reaction of the amide of formula IV with the agent capable of converting the amide IV into the imidoyl compound III is preferably carried out at temperatures of at least 0°C, for example in the range from 10°C to 85°C and preferably from 40°C to 85°C. The reaction pressure is of minor importance for the success of the reaction and thus the reaction can be conducted under reduced pressure, normal pressure (i.e. atmospheric pressure) or increased pressure. The reaction is preferably carried out in the region of atmospheric pressure, for example in the range from 0.9 to 1.2 bar. The reaction time required for the reaction is generally in the range from 2 to 20 hours, in particular from 4 to 20 hours and more preferably from 4 to 15 hours.

The reaction may in principle be carried out in substance. However, preference is given to reacting the amide IV with the agent capable of converting the amide IV into the imidoyl compound III in an organic solvent. As to the type of organic solvents suitable for this reaction, reference is made to the solvents as described hereinabove in connection with the reaction of the arylhydrazine II with the imidoyl compound III.

For the reaction, the amide IV may be brought into contact with the agent capable of converting the amide IV into the imidoyl compound III in any suitable manner. In general, the amide IV and said agent will be initially charged in a reaction vessel, if appropriate together with the solvent desired, and then the desired reaction conditions will be established. In a preferred embodiment, however, the major amount, preferably at least 80 % and more preferably the total amount or virtually the total amount (at least 95%) of amide IV may also be charged into a reaction vessel, if appropriate in the desired solvent. The major amount, preferably at least 80 % and more preferably the total amount or virtually the total amount (at least 95%) of the agent capable of converting the amide IV into the imidoyl compound III is then added under reaction conditions in the course of the reaction, if appropriate in the desired solvent.

The amide of the formula IV used in the process for the preparation of the imidoyl compounds III can in turn be obtained in a similar manner to the known prior art processes for preparing amides by reacting the compound of formula V with an amine of formula VI, for example in a similar manner to the methods as described in Organikum, 21th Ed., Wiley-VCH, Weinheim 2001, pp. 481-488.

The compounds V used to prepare the amides IV are known and processes for their preparation can be found, for example, in Organikum, 21 th Ed., Wiley-VCH, Weinheim 2001, pp. 498-499.

If not commercially available, the amine of formula VI can be obtained according to literature procedures readily available to those skilled in the art, e.g. by reductive amination of carbonyl compounds or hydrogenation of nitro compounds as described, for example, in Organikum, 21th Ed., Wiley-VCH, Weinheim 2001, pp. 584-585 and 626-629.

To react the compound V with the amine VI, the compounds will preferably be used in a molar ratio of V:Vl in the range from 1:1 to 1:10, in particular 1:2 to 1:4 and more preferably from 1:2 to 1:3.

The compound V is advantageously reacted with the amine VI at temperatures of at least 0°C, in particular in the range from 0°C to 80°C, more preferably from 10°C to 70°C and even more preferably from 40°C to 60°C. The reaction pressure is of minor importance for the success of the reaction and thus the reaction can be conducted under reduced pressure, normal pressure (i.e. atmospheric pressure) or increased pressure. The reaction is preferably carried out in the region of atmospheric pressure, for example in the range from 0.9 to 1.2 bar. The reaction time required for the reaction can be varied in a wide range and depends on a variety of factors including the capacity of heat transfer of the used equipment. In general, the reaction time is in the range from 1 to 10 hours, in particular 1 to 5 hours.

The reaction may in principle be carried out in substance. In a preferred embodiment, however, the compound V is reacted with the amine VI in an organic solvent. As to the type of organic solvents suitable for this reaction, reference is made to the solvents as described hereinabove in connection with the reaction of the arylhydrazine II with the imidoyl compound III. Moreover, protic solvents such as an alcohol or water may be used. The use of protic solvents, especially water, depends on the reactivity of the used amine VI. For example, if the amine VI is ethylamine, it is possible to use an aqueous solution of ethylamine.

For the reaction, the compound V and the amine VI may be contacted together in any suitable manner. In general, the compound V and the amine VI will be initially charged into a reaction vessel, if appropriate together with the solvent desired, and then the desired reaction conditions will be established. In a preferred embodiment, however, the major amount, preferably at least 80 % and more preferably the total amount or virtually the total amount (at least 95%) of the amine VI is placed into a reaction vessel, optionally in the desired solvent. The major amount, preferably at least 80 % and more preferably the total amount or virtually the total amount (at least 95%) of the compound V is then added to the amine VI under reaction conditions in the course of the reaction. The compound V can be added as such or preferably in the desired solvent.

A preferred embodiment of the invention relates to a process comprising the steps of
(a) reacting the compound of formula V with the amine of formula VI as described herein to obtain the amide of formula IV,
(b) reacting the amide of formula IV with an agent capable of converting the amide IV into the imidoyl compound III as described herein to obtain the imidoyl compound of formula III, and
(c) reacting the imidoyl compound of formula III with the arylhydrazine of formula II as described herein, optionally in the presence of a base, to obtain the amidrazones of formula I or a salt thereof.

Another advantage of the process according to the invention is that the amidrazones I can be obtained in a high yield and purity, without isolation of the intermediates of formulae III and IV and purification measures of these intermediates being required. Thus, in a preferred embodiment, the steps (a)+(b) or (b)+(c) or (a)+(b)+(c) as defined above are carried out in a one-pot procedure without isolating the amide of formula IV and/or the imidoyl compound of formula III. In a particularly preferred embodiment, the steps (a), (b) and (c) are carried out in a one-pot procedure without isolating the amide of formula IV and the imidoyl compound of formula III. However, it is also contemplated by this invention that the amide IV can be isolated after the step (a) by conventional methods, for example distillation, recrystallization and the like. The imidoyl compound III may also be isolated after step (b) in the same manner as above.

It is also preferred that each of the steps in the sequences (a)+(b) or (b)+(c) or (a)+(b)+(c) is carried out in the same solvent selected from those solvents described hereinabove. Preference is given to aprotic solvents, in particular aromatic hydrocarbons, halogenated aromatic hydrocarbons and heterocyclic aromatic compounds, with aromatic hydrocarbons being preferred. In a particularly preferred embodiment, toluene, chlorobenzene or pyridine is used as a solvent in each of the steps in the sequences (a)+(b) or (b)+(c) or (a)+(b)+(c), with preference being given to toluene.

If desired, the amidrazone I, the amide IV and the imidoyl compound III can be purified independently after their isolation by using techniques that are known in the art, for example by distillation, recrystallization and the like.

The overall process according to this invention has several advantages when compared to the prior art processes. The inventive process enables the introduction of the relatively expensive arylhydrazine of formula II in the very last step of the synthesis sequence, i.e. step (c). As this step is performed with a high yield and purity, the process of the present invention is highly efficient in terms of cost of goods. Moreover, the process can be conducted in ecologically friendly solvents. Since the reaction steps (a), (b) and (c) of the inventive process can be carried out in a one-pot procedure, there is no need for isolation and tedious purification of the intermediates (IV) and/or (III). Instead, it is possible to conduct any sequence of reaction steps with in situ prepared intermediates.

The advantages of the process according to the invention become particularly apparent when, in the compounds of formulae I to VI, the variables and indices are each independently defined as follows, more preferably in combination:
A in the formulae I and II denotes C-R³;
B in the formulae I and II denotes C-R⁴;
W in the formulae I and II denotes C-R⁵;
Y in the formulae I and II is halogen or C₁-C₆-haloalkyl, more preferably halogen or C₁-C₆-haloalkyl which is disposed in the 6-position;
Y in the formulae I and II is halogen, in particular halogen which is disposed in the 6-position;
Y in the formulae I and II is chlorine, in particular chlorine which is disposed in the 6-position;
n in the formulae I and II is 1;
R¹ in the formulae I, III, IV and VI is C₁-C₄-alkyl, in particular ethyl;
R² in the formulae I, III, IV and V is cyclopropyl which is unsubstituted or substituted with 1 to 3 halogen atoms and/or I to 3 C₁-C₆-alkyl groups and/or 1 to 3 C₁-C₆-haloalkyl groups;
R² in the formulae I, III, IV and V is cyclopropyl which is substituted with 2 halogen atoms and/or 1 C₁-C₆-alkyl group;
R² in the formulae I, III, IV and V is cyclopropyl which is substituted with 2 chlorine atoms and/or 1 methyl group;
R² in the formulae I, III, IV and V is a cyclopropyl group of formula VII 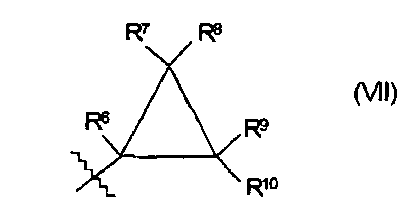 wherein R⁶ is hydrogen, C₁-C₆-alkyl or C₁-C₆-haloalkyl, and R⁷, R⁸, R⁹ and R¹⁰ are each independently selected from hydrogen, halogen, C₁-C₆-alkyl and C₁-C₆-haloalkyl;
R² in the formulae I, III, IV and V is a cyclopropyl group of formula VII wherein R⁶ is hydrogen, C₁-C₆-alkyl or C₁-C₆-haloalkyl, R⁷ and R⁸ are both halogen, and R⁹ and R¹⁰ are both hydrogen;
R² in the formulae I, III, IV and V is a cyclopropyl group of formula VII wherein R⁶ is C₁-C₆-alkyl, R⁷ and R⁸ are both halogen, and R⁹ and R¹⁰ are both hydrogen;
R² in the formulae I, III, IV and V is a cyclopropyl group of formula VII wherein R⁶ is hydrogen, methyl or trifluoromethyl, R⁷ and R⁸ are independently selected from fluoro, chloro and bromo, and R⁹ and R¹⁰ are both hydrogen;
R² in the formulae I, III, IV and V is a cyclopropyl group of formula VII wherein R⁶ is hydrogen or methyl, R⁷ and R⁸ are chloro or bromo, and R⁹ and R¹⁰ are both hydrogen;
R² in the formulae I, III, IV and V is a cyclopropyl group of formula VII wherein R⁶ is methyl, R⁷ and R⁸ are chloro or bromo, and R⁹ and R¹⁰ are both hydrogen;
R² in the formulae I, III, IV and V is a cyclopropyl group of formula VII wherein R⁶ is methyl, R⁷ and R⁸ are both chloro, and R⁹ and R¹⁰ are both hydrogen, i.e. 1-(2,2-dichloro-1-methylcydopropyl);
R³ is halogen or C₁-C₆-haloalkyl, preferably halogen, especially chlorine or bromine, with chlorine being most preferred;
R⁴ is hydrogen or halogen, especially hydrogen;
R⁵ is halogen or C₁-C₆-haloalkyl, preferably C₁-C₆-haloalkyl, especially trifluoromethyl;
X¹ in the formula III is halogen, more preferably chlorine or bromine and especially chlorine;
X² in the formula V is halogen or C₁-C₆-alkoxy;
X² in the formula V is halogen, in particular chlorine or bromine and especially chlorine;
X² in the formula V is C₁-C₆-alkoxy, preferably C₁-C₄-alkoxy, more preferably methoxy, ethoxy, propoxy, butoxy and isobutoxy, especially methoxy or ethoxy, with methoxy being most preferred;

For the process according to the invention, it has been found to be particularly advantageous when A in the formulae I and II) is C-R³ wherein R³ is halogen or C₁-C₆-haloalkyl, B in the formulae I and II is C-R⁴ wherein R⁴ is hydrogen or halogen, W in the formulae I and II is C-R⁵ wherein R⁵ is halogen or C₁-C₆-haloalkyl, Y in the formulae I and II is a halogen atom in the 6 position of the aromatic ring, n in the formulae I and II is 1, R¹ in the formulae I, III, IV and VI is C₁-C₄-alkyl, R² in the formulae I, III, IV and V is a cyclopropyl group of formula VII as defined hereinabove wherein R⁶ is hydrogen, C₁-C₆-alkyl or C₁-C₆-haloalkyl, and R⁷, R⁸, R⁹ and R¹⁰ are each independently selected from hydrogen, halogen, C₁-C₆-alkyl and C₁-C₆-haloalkyl, X¹ in the formula III is halogen, and X² in the formula V is halogen or C₁-C₆-alkoxy.

In an even more preferred embodiment, A in the formulae I and II is C-Cl, B in the formulae I and II is CH, W in the formulae I and II is C-CF₃, Y in the formulae I and II is a chlorine atom in the 6 position of the aromatic ring, n in the formulae I and II is 1, R¹ in the formulae I, III, IV and VI is ethyl, R² in the formulae I, III, IV and V is 1-(2,2-dichloro-1-methylcyclopropyl), X¹ in the formula III is chlorine, and X² in the formula V is chlorine or methoxy.

Moreover, preference is given to imidoyl compounds of formula III used as starting materials or intermediates in the process according to the invention wherein the variables R¹, R² and X¹ are each independently defined as follows, more preferably in combination:
R¹ is C₁-C₁₀-alkyl, in particular C₁-C₄-alkyl and especially ethyl;
R² is a cyclopropyl group of formula VII as defined hereinabove wherein R⁶ is hydrogen, C₁-C₆-alkyl or C₁-C₆-haloalkyl, R⁷ and R⁸ are both halogen, and R⁹ and R¹⁰ are both hydrogen, more preferably a cyclopropyl group of formula VII wherein R⁶ is C₁-C₆-alkyl, R⁷ and R⁸ are both halogen, and R⁹ and R¹⁰ are both hydrogen, even more preferably a cyclopropyl group of formula VII wherein R⁶ is hydrogen, methyl or trifluoromethyl, R⁷ and R⁸ are independently selected from fluoro, chloro and bromo, and R⁹ and R¹⁰ are both hydrogen, yet even more preferably a cyclopropyl group of formula VII wherein R⁶ is hydrogen or methyl, R⁷ and R⁸ are chloro or bromo, and R⁹ and R¹⁰ are both hydrogen, still even more preferably a cyclopropyl group of formula VII wherein R⁶ is methyl, R⁷ and R⁸ are chloro or bromo, and R⁹ and R¹⁰ are both hydrogen and especially a cyclopropyl group of formula VII wherein R⁶ is methyl, R⁷ and R⁸ are both chloro, and R⁹ and R¹⁰ are both hydrogen, i.e. 1-(2,2-dichloro-1-methylcyclopropyl);
X¹ is halogen, C₁-C₆-alkoxy, C₁-C₆-alkylsulfonate, C₁-C₆-haloalkylsulfonate, C₆-C₁₂-arylsulfonate, C₁-C₆-alkylthio, C₁-C₆-alkylphosphate or C₆-C₁₂-arylphosphate, more preferably halogen, in particular chlorine or bromine, with chlorine being preferred.

Preference is also given to imidoyl compounds of formula III wherein R¹ is C₁-C₁₀-alkyl, in particular C₁-C₄-alkyl and especially ethyl, R² is a cyclopropyl group of formula VII as defined hereinabove wherein R⁶ is hydrogen, C₁-C₆-alkyl or C₁-C₆-haloalkyl, R⁷ and R⁸ are both halogen, R⁹ and R¹⁰ are both hydrogen, and X¹ is halogen, in particular chlorine or bromine, with chlorine being preferred.

Preference is also given to imidoyl compounds of formula III wherein R¹ is C₁-C₁₀-alkyl, in particular C₁-C₄-alkyl and especially ethyl, R² is a cyclopropyl group of formula VII as defined hereinabove wherein R⁶ is C₁-C₆-alkyl, R⁷ and R⁸ are both halogen, and R⁹ and R¹⁰ are both hydrogen, and X¹ is halogen, in particular chlorine or bromine, with chlorine being preferred.

Preference is also given to imidoyl compounds of formula III wherein R¹ is C₁-C₁₀-alkyl, in particular C₁-C₄-alkyl and especially ethyl, R² is a cyclopropyl group of formula VII as defined hereinabove wherein R⁶ is hydrogen, methyl or trifluoromethyl, R⁷ and R⁸ are independently selected from fluoro, chloro and bromo, and R⁹ and R¹⁰ are both hydrogen, and X¹ is halogen, in particular chlorine or bromine, with chlorine being preferred.

Preference is also given to compounds of formula III wherein R¹ is C₁-C₁₀-alkyl, in particular C₁-C₄-alkyl and especially ethyl, R² is a cyclopropyl group of formula VII as defined hereinabove wherein R⁶ is hydrogen or methyl, R⁷ and R⁸ are independently selected from chloro and bromo, and R⁹ and R¹⁰ are both hydrogen, and X¹ is halogen, especially chlorine or bromine, with chlorine being preferred.

Preference is also given to compounds of formula III wherein R¹ is C₁-C₁₀-alkyl, in particular C₁-C₄-alkyl and especially ethyl, R² is 1-(2,2-dibromo-1-methylcyclopropyl) or 1-(2,2-dichloro-1-methylcyclopropyl) and X¹ is halogen, especially chlorine or bromine, with chlorine being preferred.

Particularly preferred is a compound of formula III wherein R¹ is ethyl, R² is 1-(2,2-dichloro-1-methylcyclopropyl) and X¹ is chlorine, i.e. 2,2-Dichloro-N-ethyl-1-methylcyclopropanecarboximidoyl chloride (see formula III' below).

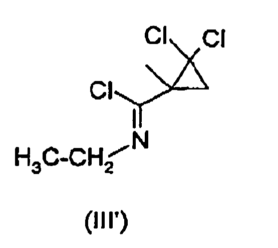

This invention also relates to the use of the imidoyl compound of formula III (and especially compound III') as a starting material or an intermediate for the preparation of amidrazones or a salt thereof, in particular the amidrazones of formula I or a salt thereof.

Also, preference is given to amides of formula IV used as starting materials or intermediates in the process according to the invention wherein the variables R¹ and R² are each independently defined as follows, more preferably in combination:
R¹ is C₁-C₁₀-alkyl, in particular C₁-C₄-alkyl and especially ethyl;
R² is a cyclopropyl group of formula VII wherein R⁶ is methyl, R⁷ and R⁸ are chloro or bromo, and R⁹ and R¹⁰ are both hydrogen and especially a cyclopropyl group of formula V11 wherein R⁶ is methyl, R⁷ and R⁶ are both chloro, and R⁹ and R¹⁰ are both hydrogen, i.e. 1-(2,2-dichloro-1-methylcyclopropyl).

Preference is also given to amides of formula IV wherein R¹ is C₁-C₁₀-alkyl, in particular C₁-C₄-alkyl and especially ethyl and R² is 1-(2,2-dibromo-1-methylcyclopropyl) or 1-(2,2-dichloro-1-methylcyclopropyl).

Particularly preferred is an amide of formula IV wherein R¹ is ethyl and R² is 1-(2,2-dichloro-1-methylcyclopropyl), i.e. 2,2-dichloro-1-methyl-cyclopropane-carboxylic-acid ethylamide (see formula IV' below).

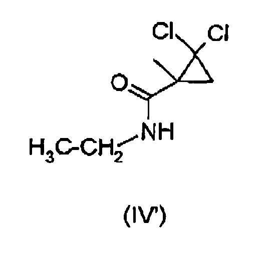

This invention also relates to the use of the amide of formula IV (and especially compound IV') as a starting material or an intermediate for the preparation of amidrazones or a salt thereof, in particular the amidrazones of formula I or a salt thereof.

Although the imidoyl compound III and the amide IV are useful starting materials or intermediates for the preparation of amidrazones, their use as starting materials or intermediates for the preparation of other products likewise forms part of the scope of protection of this invention.

The process according to the invention will be illustrated, in case of preparing 2,2-dichloro-N'-(2,6-dichloro-4-trifluoromethylphenyl)-N-ethyl-1-methylcyclopropane-carbohydrazonamide (I') via the intermediates (III') and (IV'), by the following reaction scheme:

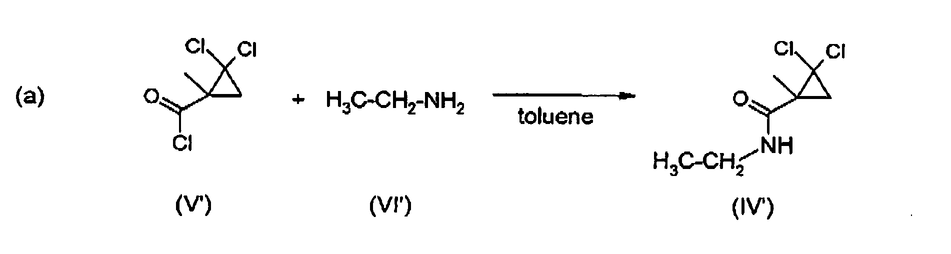

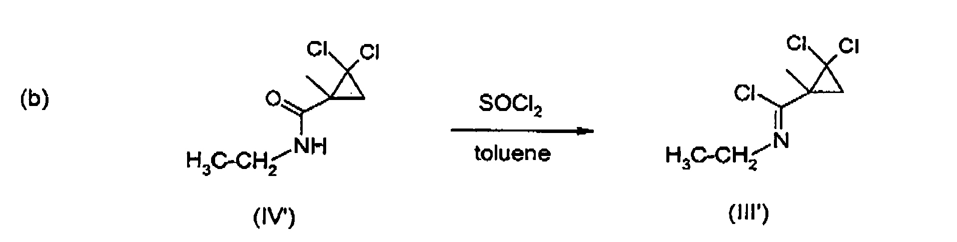

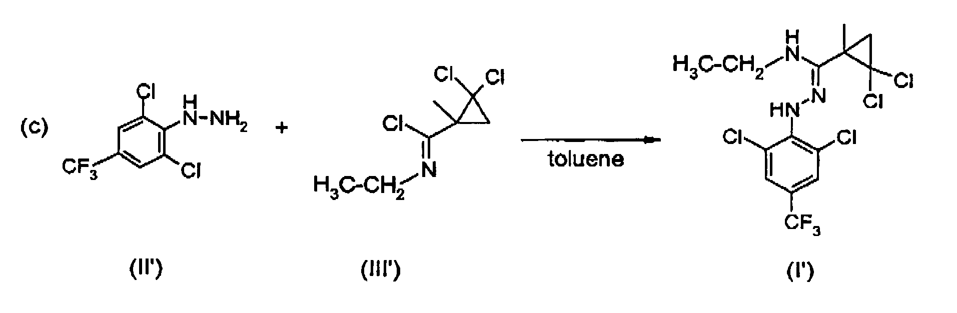

In addition to the general methods described above for the preparation of the compounds of formula V, the compounds V wherein R² is a dichlorocyclopropyl group (e.g. 2,2-dichloro-1-methylcyclopropyl) may also be prepared by the following procedure: (1) reaction of dichlorocarbene with conjugated dienes, e.g. with 2-methyl-1,3-butadiene (isoprene), to give the corresponding addition product (e.g. 2,2-dichloro-1-methyl-1-vinylcylopropane), (2) oxidation of the addition product with potassium permanganate in acetone at 0°C to yield the corresponding carboxylic acid (e.g. 2,2-dichloro-1-methylcyclopropane-1-carboxylic acid) and (3) conversion of the carboxylic acid into the corresponding acid chloride, e.g. with SOCl₂/benzene. This sequence of reaction steps (1), (2) and (3) has been reported in Toke, S. M. and Kulkarni, G. H., Indian Journal of Chemistry, Vol. 30B, January 1991, pp. 82-84. The sequence of steps (1) and (2) can also be found in M. Orchin and E. C. Herrick, J. Org. Chem., 24 (1959), pp. 139-140. The carboxylic acid used in step (3) hereinabove can also be obtained by dichlorocarbene addition to metharylic acid esters and subsequent ester saponification as described in GB-A 1323183.

The examples below serve merely to illustrate the invention and are not to be interpreted in a restrictive manner.

The purities reported were determined by means of gas chromatography (GC) or high performance liquid chromatography (HPLC) via the area ratios of the particular peaks.

In connection with the NMR spectra, s is a singlet, d is a doublet, t is a triplet and q is a quartet. MS stands for mass spectrum and IR for IR spectrum.

### Example 1: Preparation of 2,2-Dichloro-1-methyl-cyclopropanecarbonyl chloride (V')

2,2-Dichloro-1-methyl-cyclopropanecarboxylic acid (1002 g) was charged into a 4 L vessel and dissolved by addition of dichloromethane (1836 g) The solution was warmed up to 40°C. Dimethylformamide (0.8 g) was added to the solution. Thionylchloride (1411 g) was added over 3 h at 40°C. The acidic off-gas was vented through a sodium hydroxide solution. The reaction mixture was kept at 40°C over about 12 h. The solvent was removed at 40°C and a final vacuum of 25 mbar. The crude oil was rectifiied at 10-20 mbar. The fractions between 60 and 74°C were collected. The rectification was repeated at 25 mbar after colorization of the product occurred during storage. The fractions between 77-78°C were collected. A total mass of 771 g of the title compound with a GC purity of 97-98 area% was obtained. This corresponds to a yield of 68 %.
MS (EI): 186 m/e (M⁺-ion), 151 (M⁺-Cl)
IR (KBr): 1788 cm⁻¹ (C=O)
¹H-NMR (500 MHz, CDCl₃): δ/ppm = 1.65 (d, 1H); 1.75 (s, 3H); 2.38 (s, 1H)
¹³C-NMR (500 MHz, CDCl₃): δ/ppm = 19.28 (q); 32.41 (t); 43.71 (s); 61.85 (s); 171.18 (s)

### Example 2: Preparation of 2,2-Dichloro-1-methyl-cyclopropanecarboxylic-acid ethylamide (IV')

2,2-Dichloro-1-methyl-cyclopropanecarbonyl chloride (25 g) was dosed to pre-charged aqueous ethylamine (24.5 g 70 wt-% solution) at 0°C to 10°C in a 250 ml vessel. A white precipitate was formed which was dissolved by addition of 80 ml toluene at 50°C. The aqueous phase was separated and the toluene was removed by distillation. A total mass of 23.5 g of the title compound was obtained as a white solid. This corresponds to a yield of 96%.
melting point: 101.5-103.3°C
MS (El): 195 m/e (M⁺-ion)
IR (KBr): 3321 cm-¹ (N-H), 1641 cm⁻¹ (acid amide 1), 1538 cm⁻¹ (acid amide 2)
¹H-NMR (500 MHz, CDCl₃): δ/ppm = 1.18 (t, 3H); 1.38 (d, 1H); 1.62 (s, 3H); 2.20 (d, 1 H); 3.28 - 3.48 (m, 2H); 6.44 (s broad, 1H)
¹³C-NMR (500 MHz, CDCl₃): δ/ppm = 14.75 (q); 19.57 (q); 30.40 (t); 35.14 (t); 36.44 (s); 62.57 (s); 168.16 (s)

### Example 3: Preparation of 2,2-Dichloro-N-ethyl-1-methyl-cyclopropanecarboximidoyl chloride (III')

2,2-Dichloro-1-methyl-cyclopropanecarboxylic-acid ethylamide (99.3 g), toluene (500 g) and dimethylformamide (0.5 g) were charged into a 1 L vessel. The mixture was warmed up to 40°C and became a solution. Thionylchloride (91.3 g) was added over 30 min at 40°C. The solution was heated up to 110°C (reflux) and kept for 6 h at reflux. The acidic off-gas was vented through a sodium hydroxide solution. The solvent was removed at 50°C and a final vacuum of 100 mbar. The concentrated solution was further rectified at 4 mbar. The fractions between 60.4 - 63.4°C were collected. A total mass of 87 g of the title compound with a GC purity of 98 - 99 area% was obtained. This corresponds to a yield of 80 %.
MS (EI): 213 m/a (M⁺-ion)
IR (KBr): 1691 cm⁻¹ (C=N)
¹H-NMR (500 MHz, CDCl₃): δ/ppm = 1.17 (t, 3H); 1.52 (d, 1H); 1.65 (s, 3H); 2.30 (d, 1H); 3.54 (q, 2H)
¹³C-NMR (500 MHz, CDCl₃): δ/ppm = 14.20 (q); 19.70 (q); 32.66 (t); 40.25 (s); 47.83 (t); 63.44 (s); 143.42 (s)

### Example 4: Preparation of 2,2-Dichloro-N'-(2,6-dichloro-4-trifluoromethylphenyl)-N-ethyl-1-methylcyclopropane-carbohydrazonamide (I')

2,2-Dichloro-4-trifluoromethylphenyl-hydrazine (22.6 g), tri-n-butylamine (20.7 g) and toluene (15.2 g) were charged into a 250 ml vessel. The mixture was heated up to 70°C. A solution of 2,2-Dichloro-N-ethyl-1-methyl-cyclopropanecarboximidoyl chloride (20.0 g) in toluene (15.2 g) were added over 2 h at 70°C. The reaction mixture was kept at 70°C for 4 h after addition. Then water (20 g) and 1 molar hydrochloric acid (20 g) were added to the reaction mixture. After phase separation the toluene was evaporated at reduced pressure at 50°C. The residual oil (46.6 g) was dissolved in isopropanole (80 g) and crystallized by addition of water (80 g). The solid product was dried at 30°C /2 mbar in a vacuum dryer. A total mass of 33.8 g of the title compound with a HPLC purity of 99.9 area-% was obtained a white solid. This corresponds to a yield of 85%.
melting point: 79°C
MS (EI): 421 m/e (M⁺-ion, isotopic pattern corresponds to 6 Cl atoms)

The ¹H and ¹³C NMR spectra of the isolated compound showed two sets of signals, referred to hereinafter as "isomer 1" and "isomer 2".
¹H-NMR (400 MHz, DMSO-d6): δ/ppm =1.1 (t, 3H, isomer 1); 1.28 (t, 3H, isomer 2); 1.47 (s, 3H, isomer 2); 1.58 (s. 3H, isomer 1); 1.6 (d, 1H, isomer 2); 1.7 (d, 1H, isomer 1); 2.0 (d, 1H, isomer 1); 2.1 (d, 1H, isomer 2); 2.9 -3.18 (m, 2H, isomer 1); 3.15 - 3.43 (m, 2H, isomer 2); 6.3 - 6.45 (m, 1H isomer 1 and 2); 6.95 (s, 1H, isomer 2); 7.2 (s, 1H, isomer 1); 7.73 (s, 2H, isomer 1 and 2)
¹³C-NMR (500 MHz, DMSO-d6): δ/ppm = 14.00 (q, isomer 1); 16.07 (q, isomer 2); 18.58 (q, isomer 1); 20.30 (q, isomer 2); 30.19 (t, isomer 2); 30.32 (t, isomer 1); 33.15 (s, isomer 2); 33.88 (s, isomer 1); 35.97 (t, isomer 1); 37.88 (t, isomer 2); 64.37 (s, isomer 1); 65.43 (s, isomer 2); 121.44 (CF3, isomer 1); 121.94 (CF3, isomer 2); 124.2 - 126.37 (aromatic ring C-atoms of isomer 1 and 2); 144.14 (s, isomer 1); 144.99 (s, isomer 2); 152.87 (s, isomer 2); 156.16 (s, isomer 1)

### Example 5: Preparation of 2,2-Dichloro-N'-(2,6-dichloro-4-trifluoromethylphenyl)-N-ethyl-1-methylcyclopropane-carbohydrazonamide (I')

2,2-Dichloro-4-trifluoromethylphenyl-hydrazine (100 g), tri-n-butylamine (76.4 g) and toluene (104.1 g) were charged into a 750 ml vessel. The mixture was heated up to 45°C. 186.3 g of a solution of 48.4.wt-% of 2,2-Dichloro-N-ethyl-1-methylcyclopropanecarboximidoyl chloride in toluene were then added over a period of 2 h while keeping the reaction mixture at 45°C. After addition the reaction mixture was kept at 70°C for 1 h. After cooling to 25°C water (190 g) was added and the pH was adjusted to 4 by addition of sodium hydroxide solution. After phase separation the toluene phase was extracted twice with water (2 x 125g) and then evaporated under reduced pressure at 50°C. The residual oil was dissolved in isopropanole (254 g) and crystallized by addition of water (280 g). The solid product was dried at 30°C / 2 mbar in a vacuum dryer. A total mass of 158.6 g of the title compound with a HPLC purity of 99.9 area-% was obtained as a white solid. This corresponds to a yield of 92%.
melting point: 79°C
MS (El): 421 m/e (M⁺-ion, isotopic pattern corresponds to 6 Cl atoms)

### Examples 6: Preparation of 2,2-Dichloro-N-ethyl-1-methyl-cyclopropanecarboximidoyl chloride (III') without isolation of the intermediates

2,2-Dichloro-1-methyl-cyclopropanecarboxylic acid (450 g) was charged into a 4 L vessel as a melt at 80°C and dissolved by addition of toluene (1061 g) at 80°C. Thionylchloride (412 g) was added over 1 h at 80°C. The acidic off-gas was vented through a sodium hydroxide solution. A part of the solvent (approx. 200 g) was stripped off together with the off-gas. The reaction mixture was kept at 80-85°C over 4 h. A part of the solvent (approx. 435 g) was removed by distillation at 110°C head temperature. The concentrated solution (approx. 50 wt-% of acid chloride) was dosed during 45 min to pre-charged mixture of aqueous ethylamine (481.5 g of a 70 wt-% solution) and toluene (490 g) at 40°C in a 4 L vessel. Cooling was applied in order to maintain the temperature at 40°C. The reaction mixture was stirred for 1 h after end of the dosage. The phases were separated at 40°C. The toluene layer was extracted once with water (250 g) and dried by azeotropic distillation of toluene/water (454 g) at 110°C head temperature. The solution (approx. 50 wt-% of acid ethyl amide) was diluted with toluene (1860 g) and kept at 100°C. Dimethylformamide (2.6 g) was added to the solution. Thionylchloride (468 g) was added over 20 min at 100°C. The acidic off-gas was vented through a sodium hydroxide solution. After 6 h at 100°C additional thionylchloride (38 g) was added. The reaction mixture was kept at 100°C for further 8 h. The reaction solution was concentrated by distillation at 110°C head temperature to a residual mass of 1184 g. This solution was further concentrated at 50 mbar and then rectified at 4 mbar. The fractions between 65.4 and 68.5°C were collected. A total mass of 429 g of the title compound with a GC purity of 98-99 area% was obtained. This corresponds to a yield of 76 % based on the used acid.

The reaction solution obtained in Example 6 can also be used to prepare 2,2-Dichloro-N'-(2,6-dichloro-4-trifluoromethylphenyl)-N-ethyl-1-methylcyclopropane-carbohydrazonamide (I') according to the procedure described in Example 4 or 5.

## Claims

1. A process for preparing amidrazones of formula I or a salt thereof 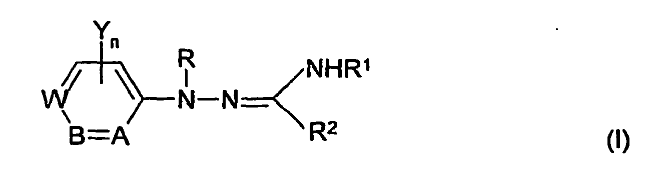 wherein
R is hydrogen;
R¹ is C₁-C₁₀-alkyl;
R² is C₃-C₁₀-cycloalkyl which is unsubstituted or substituted with 1 to 5 halogen atoms and/or 1 to 3 C₁-C₆-alkyl groups and/or 1 to 3 C₁-C₆-haloalkyl groups;
A is C-R³ or N;
B is C-R⁴ or N;
W is C-R⁵ or N;
with the proviso that one of A, B and W is other than N;
R3, R4, R⁵ are each independently hydrogen, halogen, nitro, cyano, amino, mercapto, hydroxy, C₁-C₁₀-alkyl, C₂-C₁₀-alkenyl, C₂-C₁₀-alkynyl, C₃-C₆-cycloalkyl, C₁-C₆-alkoxy, C₁-C₆-alkylamino, di(C₁-C₆-alkyl)- amino, C₁-C₆-alkylthio, C₁-C₆-alkylsulfonyl, C₁-C₆-alkylsulfinyl or a 5- to 6-membered aromatic ring which may contain 1 to 4 hetero- atoms selected from oxygen, sulfur and nitrogen;
Y is hydrogen, halogen, cyano, nitro, amino, hydroxy, mercapto, C₁-C₆-alkyl, C₂-C₁₀-alkenyl, C₂-C₁₀-alkynyl, C₃-C₆-cycloalkyl, C₁₋C₆-alkoxy, C₁-C₆-alkylamino, di(C₁₋C₆)-alkylamino, C₁-C₆-alkylthio, C₁-C₆-alkylsulfonyl, or C₁-C₆-alkylsulfinyl; and
n is 0, 1, or 2,
comprising reacting an arylhydrazine of formula II 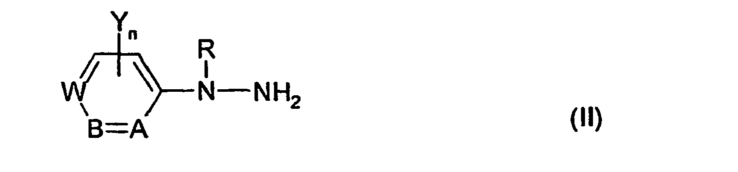 wherein R, A, B, W, Y and n are each as defined above,
with an imidoyl compound of formula III 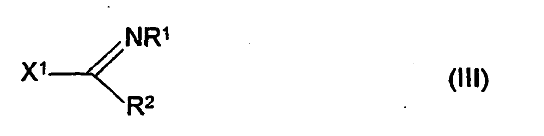 wherein R¹ and R² are each as defined above and X¹ is halogen, C₁-C₆-alkoxy, C₁-C₆-alkylsulfonate, C₁-C₆-haloalkylsulfonate, C₆-C₁₂-arylsulfonate, C₁-C₆-alkylthio, C₁-C₆-alkylphosphate, or C₆-C₁₂-arylphosphate, optionally in the presence of a base.

2. The process according to claim 1 wherein the reaction of the arylhydrazine of formula II with the imidoyl compound of formula III is carried out in the presence of a base to obtain the amidrazone of formula I.

3. The process according to claim 2 wherein the base is a tertiary amine.

4. The process according to claim 3 wherein the tertiary amine is tri-n-butylamine.

5. The process according to any of claims 2 to 4 wherein the resulting amidrazone of formula I is subsequently reacted with a suitable acid to form an acid addition salt of said amidrazone.

6. The process according to any of claims 1 to 5 wherein the imidoyl compound of formula III is obtained by reacting an amide of formula IV 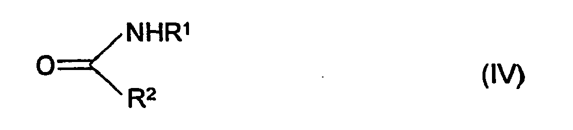 wherein R¹ and R² are each as defined above,
with an agent capable of converting the amide IV into the imidoyl compound III.

7. The process according to claim 6 wherein the agent capable of converting the amide IV into the imidoyl compound III is selected from halogenating agents, O-alkylating agents, phosphating agents, and sulfonylating agents.

8. The process according to claim 6 or 7 wherein the amide of formula IV is obtained by reacting a compound of formula V 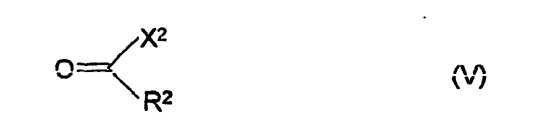 wherein X² is a suitable leaving group and R² is as defined above,
with an amine of formula VI
R¹-NH₂ (VI)
wherein R¹ is as defined above.

9. The process according to any of claims 1 to 8 comprising the steps of
(a) reacting the compound of formula V with the amine of formula VI to obtain the amide of formula IV,
(b) reacting the amide of formula IV with the agent capable of converting the amide of formula IV into the imidoyl compound of formula III to obtain said imidoyl compound, and
(c) reacting the imidoyl compound of the formula III with an arylhydrazine of formula II, optionally in the presence of a base, to obtain the amidrazone of formula I or a salt thereof.

10. The process according to claim 9 wherein the steps (a), (b) and (c) are carried out in a one-pot procedure without isolating the amide of formula IV and the imidoyle compound of formula III.

11. The process according to claim 9 or 10 wherein each of the steps (a), (b) and (c) is carried out in the same solvent.

12. The process according to claim 11 wherein the solvent is an aromatic hydrocar bon.

13. The process according to any of claims 1 to 12 wherein R² in the formulae I, III, IV and V is cyclopropyl which is substituted with 2 halogen atoms and/or 1 C₁-C₆-alkyl group.

14. The process according to any of claims 1 to 13 wherein R² in the formulae I, III, IV and V is a cyclopropyl group of formula VII 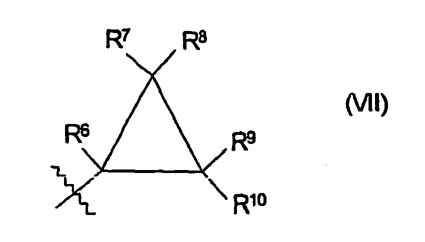 wherein R⁶ is hydrogen, C₁-C₆-alkyl or C₁-C₆-haloalkyl, and R⁷, R⁸, R⁹ and R¹⁰ are each independently selected from hydrogen, halogen, C₁-C₆-alkyl or C₁-C₆-haloalkyl.

15. The process according to claim 14 wherein R⁶ is C₁-C₆-alkyl, R⁷ and R⁸ are both halogen, and R⁹ and R¹⁰ are both hydrogen.

16. The process according to any of claims 1 to 15 wherein R² in the formulae I, III, IV and V is 1-(2,2-dichloro-1-methylcyclopropyl).

17. The process according to any of claims 1 to 16 wherein A in the formulae I and II denotes C-R³ wherein R³ is halogen or C₁-C₆-haloalkyl, B in the formulae I and II denotes C-R⁴ wherein R⁴ is hydrogen or halogen, W in the formulae I and II denotes C-R⁵ wherein R⁵ is halogen or C₁-C₆-haloalkyl, Y in the formulae I and II is a halogen atom in the 6 position of the aromatic ring, n in the formulae I and II is 1, R¹ in the formulae I, III, IV and VI is C₁-C₄-alkyl, X¹ in the formula III is halogen, and X² in the formula V is halogen or C₁-C₆-alkoxy.

18. The process according to any of claims 1 to 17 wherein A in the formulae I and II is C-Cl, B in the formulae I and II is CH, W in the formulae I and II is C-CF₃, Y in the formulae I and II is a chlorine atom in the 6 position of the aromatic ring, n in the formulae I and II is 1, R¹ in the formulae I, III, IV and VI is ethyl, X¹ in the formula III is chlorine, and X² in the formula V is chlorine or methoxy.

19. An imidoyl compound of formula III 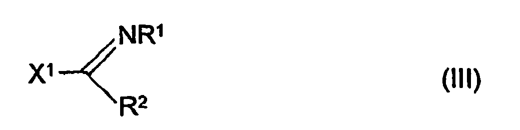 wherein
R¹ is C₁-C₁₀-alkyl, C₃-C₁₀-alkenyl, C₃-C₁₀-alkynyl, C₃-C₁₂-cycloalkyl, di(C₁-C₆- alkyl)-amino, C₁-C₆-alkylsulfonyl or C₁-C₆-alkylsulfinyl, wherein in each of the above radicals the alkyl, alkenyl, alkynyl or cycloalkyl groups are unsubsti- tuted or substituted with 1 to 3 substituents independently selected from halogen, nitro, cyano, C₁-C₆-alkoxy, C₁-C₆-haloalkoxy, C₁-C₆-alkylthio, C₁-C₆-haloalkylthio, C₁-C₆-alkylsulfonyl, C₁-C₆-alkylsulfinyl, C₁-C₆-haloalkylsulfonyl, C₁-C₆-haloalkylsulfinyl, C₃-C₆-cycloalkyl, phenyl and pyridyl;
R² is cyclopropyl which is substituted with 1 to 3 halogen atoms and/or 1 to 3 C₁-C₆-alkyl groups and/or 1 to 3 C₁-C₆-haloalkyl groups; and
X¹ is halogen, C₁-C₆-alkoxy, C₁-C₆-alkylsulfonate, C₁-C₆-haloalkylsulfonate, C₆-C₁₂-arylsulfonate, C₁-C₆-alkylthio, C₁-C₆-alkylphosphate, or C₆-C₁₂-arylphosphate.

20. The imidoyl compound of formula III according to claim 19 wherein R¹ is C₁-C₁₀-alkyl, R² is cyclopropyl which is substituted with 2 halogen atoms and/or 1 C₁-C₆-alkyl group and X¹ is halogen.

21. The imidoyl compound of formula III according to claim 19 or 20 wherein R² is a cyclopropyl group of formula VI I as defined above wherein R⁶ is C₁-C₆-alkyl, R⁷ and R⁸ are both halogen, and R⁹ and R¹⁰ are both hydrogen.

22. 2,2-Dichloro-N-ethyl-1-methyl-cyclopropanecarboximidoyl chloride of the formula III'

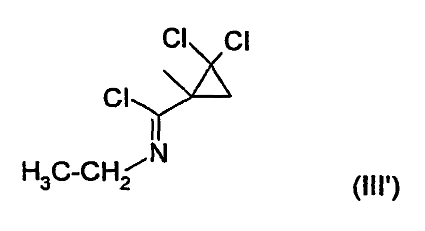

23. An amide of formula IV 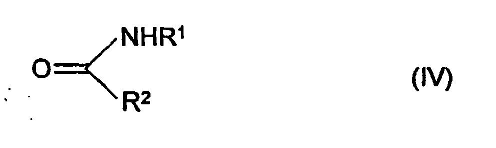 wherein
R¹ is C₁-C₁₀-alkyl, C₃-C₁₀-alkenyl, C₃-C₁₀-alkynyl, C₃-C₁₂-cycloalkyl, di(C₁-C₆- alkyl)-amino, C₁-C₆-alkylsulfonyl, or C₁-C₆-alkylsulfinyl, wherein in each of the above radicals the alkyl, alkenyl, alkynyl or cycloalkyl groups are unsubsti- tuted or substituted with 1 to 3 substituents independently selected from halogen, nitro, cyano, C₁-C₆-alkoxy, C₁-C₆-haloalkoxy, C₁-C₆-alkylthio, C₁-C₆-haloalkylthio, C₁-C₆-alkylsulfonyl, C₁-C₆-alkylsulfinyl, C₁-C₆-haloalkylsulfonyl, C₁-C₆-haloalkylsulfinyl and C₃-C₆-cycoalkyl; and
R² is a cyclopropyl group of formula VII as defined above wherein R⁶ is C₁-C₆ al- kyl, R⁷ and R⁸ are both chlorine, and R⁹ and R¹⁰ are both hydrogen.

24. The amide of formula IV according to claim 23 wherein R¹ is C₁-C₄-alkyl.

25. 2,2-dichloro-1-methyl-cyclopropane-carboxylic-acid ethylamide of the formula IV'

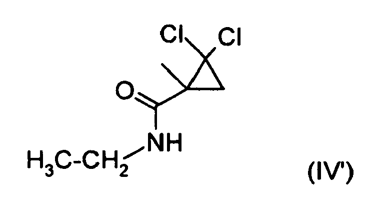

## Patentansprüche

1. Verfahren zur Herstellung von Amidrazonen der Formel I oder einem Salz davon 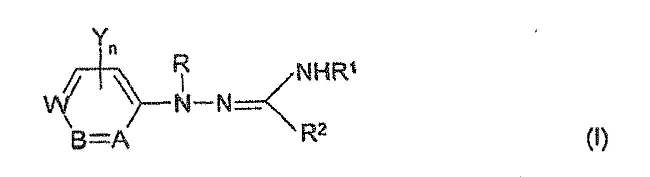 worin
R für Wasserstoff steht;
R¹ für C₁-C₁₀-Alkyl steht;
R² für C₃-C₁₀-Cycloalkyl, das gegebenenfalls durch 1 bis 5 Halogenatome und/oder 1 bis 3 C₁-C₆- Alkylgruppen und/oder 1 bis 3 C₁-C₆-Halogen- alkylgruppen substituiert ist, steht;
A für C-R³ oder N steht;
B für C-R⁴ oder N steht;
W für C-R⁵ oder N steht;
mit der Maßgabe, daß mindestens eine der Gruppen A, B und W nicht für N steht;
R³, R⁴ und R⁵ jeweils unabhängig voneinander für Wasserstoff, Halogen, Nitro, Cyano, Amino, Mercapto, Hydroxy, C₁-C₁₀-Alkyl, C₂-C₁₀- Alkenyl, C₂-C₁₀-Alkinyl, C₃-C₆-Cycloalkyl, C₁- C₆-Alkoxy, C₁-C₆-Alkylamino, Di (C₁-C₆-alkyl)- amino, C₁-C₆-Alkylthio, C₁-C₆-Alkylsulfonyl, C₁-C₆-Alkylsulfinyl oder einen 5- bis 6- gliedrigen aromatischen Ring, der 1 bis 4 unter Sauerstoff, Schwefel und Stickstoff ausgewählte Heteroatome enthalten kann, stehen;
Y für Wasserstoff, Halogen, Cyano, Nitro, Amino, Hydroxy, Mercapto, C₁-C₆-Alkyl, C₂-C₁₀-Alkenyl, C₂-C₁₀-Alkinyl, C₃-C₆-Cycloalkyl, C₁-C₆-Alkoxy, C₁-C₆-Alkylamino, Di(C₁-C₆-alkyl)amino, C₁-C₆- Alkylthio, C₁-C₆-Alkylsulfonyl oder C₁-C₆-Alkyl- sulfinyl steht und
n für 0, 1 oder 2 steht,
bei dem man ein Arylhydrazin der Formel II 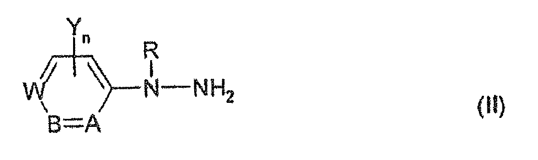 worin R, A, B, W, Y und n jeweils die oben angegebene Bedeutung besitzen,
mit einer Imidoylverbindung der Formel III 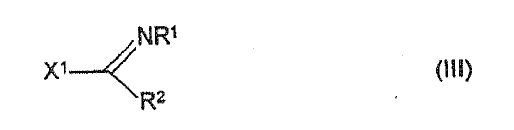 worin R¹ und R² jeweils die oben angegebene Bedeutung besitzen und X¹ für Halogen, C₁-C₆-Alkoxy, C₁-C₆-Alkylsulfonat, C₁-C₆-Halogenalkylsulfonat, C₆-C₁₂-Arylsulfonat, C₁-C₆-Alkylthio, C₁-C₆-Alkylphosphat oder C₆-C₁₂-Arylphosphat steht, umsetzt, gegebenenfalls in Gegenwart einer Base.

2. Verfahren nach Anspruch 1, bei dem man die Umsetzung des Arylhydrazins der Formel II mit der Imidoylverbindung der Formel III in Gegenwart einer Base durchführt, wobei man das Amidrazon der Formel I erhält.

3. Verfahren nach Anspruch 2, bei dem es sich bei der Base um ein tertiäres Amin handelt.

4. Verfahren nach Anspruch 3, bei dem es sich bei dem tertiären Amin um Tri-n-butylamin handelt.

5. Verfahren nach einem der Ansprüche 2 bis 4, bei dem man das resultierende Amidrazon der Formel I anschließend mit einer geeignete Säure umsetzt, wobei man ein Säureadditionssalz des Amidrazons erhält.

6. Verfahren nach einem der Ansprüche 1 bis 5, bei dem man die Imidoylverbindung der Formel III durch Umsetzung eines Amids der Formel IV 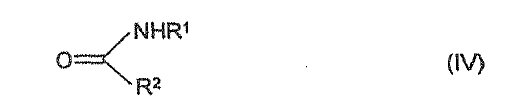 worin R¹ und R² jeweils die oben angegebene Bedeutung besitzen,
mit einem Mittel, das zur Umwandlung des Amids IV in die Imidoylverbindung III befähigt ist, erhält.

7. Verfahren nach Anspruch 6, bei dem man das Mittel, das zur Umwandlung des Amids IV in die Imidoylverbindung III befähigt ist, unter Halogenierungsmitteln, O-Alkylierungsmitteln, Phosphatierungsmitteln und Sulfonylierungsmitteln auswählt.

8. Verfahren nach Anspruch 6 oder 7, bei dem man das Amid der Formel IV durch Umsetzung einer Verbindung der Formel V 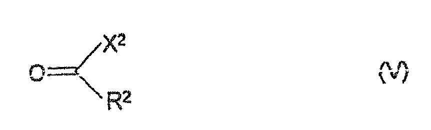 worin X² für eine geeignete Abgangsgruppe steht und R² die oben angegebene Bedeutung besitzt,
mit einem Amin der Formel VI
R¹-NH₂ (VI)
worin R¹ die oben angegebene Bedeutung besitzt, erhält.

9. Verfahren nach einem der Ansprüche 1 bis 8, bei dem man
(a) die Verbindung der Formel V mit dem Amin der Formel VI umsetzt, wobei man das Amid der Formel IV erhält,
(b) das Amid der Formel IV mit dem Mittel, das zur Umwandlung des Amids der Formel IV in die Imidoylverbindung der Formel III befähigt ist, umsetzt, wobei man die Imidoylverbindung erhält, und
(c) die Imidoylverbindung der Formel III mit einem Arylhydrazin der Formel II umsetzt, gegebenenfalls in Gegenwart einer Base, wobei man das Amidrazon der Formel I oder ein Salz davon erhält.

10. Verfahren nach Anspruch 9, bei dem man die Schritte (a), (b) und (c) im Eintopfverfahren ohne Isolierung des Amids der Formel IV und der Imidoylverbindung der Formel III durchführt.

11. Verfahren nach Anspruch 9 oder 10, bei dem man jeden der Schritte (a), (b) und (c) in dem gleichen Lösungsmittel durchführt.

12. Verfahren nach Anspruch 11, bei dem es sich bei dem Lösungsmittel um einen aromatischen Kohlenwasserstoff handelt.

13. Verfahren nach einem der Ansprüche 1 bis 12, bei dem R² in den Formeln I, III, IV und V für Cyclopropyl, das durch 2 Halogenatome und/oder 1 C₁-C₆-Alkylgruppe substituiert ist, steht.

14. Verfahren nach einem der Ansprüche 1 bis 13, bei dem R² in den Formeln I, III, IV und V für eine Cyclopropylgruppe der Formel VII 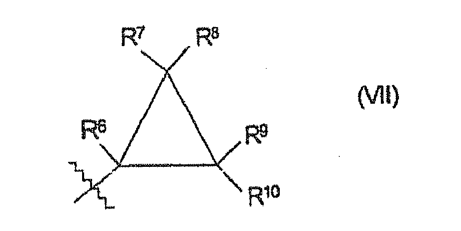 worin R⁶ für Wasserstoff, C₁-C₆-Alkyl oder C₁-C₆-Halogenalkyl steht und R⁷, R⁸, R⁹ und R¹⁰ jeweils unabhängig voneinander unter Wasserstoff, Halogen, C₁-C₆-Alkyl oder C₁-C₆-Halogenalkyl ausgewählt sind, steht.

15. Verfahren nach Anspruch 14, bei dem R⁶ für C₁-C₆-Alkyl steht, R⁷ und R⁸ beide für Halogen stehen und R⁹ und R¹⁰ beide für Wasserstoff stehen.

16. Verfahren nach einem der Ansprüche 1 bis 15, bei dem R² in den Formeln I, III, IV und V für 1-(2,2-Dichlor-1-methylcyclopropyl) steht.

17. Verfahren nach einem der Ansprüche 1 bis 16, bei dem A in den Formeln I und II C-R³ bedeutet, worin R³ für Halogen oder C₁-C₆-Halogenalkyl steht, B in den Formeln I und II für C-R⁴ steht, worin R⁴ für Wasserstoff oder Halogen steht, W in den Formeln I und II für C-R⁵ steht, worin R⁵ für Halogen oder C₁-C₆-Halogenalkyl steht, Y in den Formeln I und II für ein Halogenatom in der 6-Position des aromatischen Rings steht, n in den Formeln I und II für 1 steht, R¹ in den Formeln I, III, IV und VI für C₁-C₄-Alkyl steht, X¹ in der Formel III für Halogen steht und X² in der Formel V für Halogen oder C₁-C₆-Alkoxy steht.

18. Verfahren nach einem der Ansprüche 1 bis 17, bei dem A in den Formeln I und II für C-Cl steht, B in den Formeln I und II für CH steht, W in den Formeln I und II für C-CF₃ steht, Y in den Formeln I und II für ein Chloratom in der 6-Position des aromatischen Rings steht, n in den Formeln I und II für 1 steht, R¹ in den Formeln I, III, IV und VI für Ethyl steht, X¹ in der Formel III für Chlor steht und X² in der Formel V für Chlor oder Methoxy steht.

19. Imidoylverbindung der Formel III 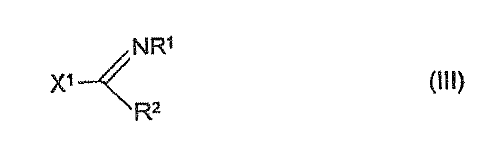 worin
R¹ für C₁-C₁₀-Alkyl, C₃-C₁₀-Alkenyl, C₃-C₁₀-Alkinyl, C₃-C₁₂-Cycloalkyl, Di(C₁-C₆-alkyl)amino, C₁-C₆- Alkylsulfonyl oder C₁-C₆-Alkylsulfinyl steht, wobei in jedem der obigen Reste die Alkyl-, Alkenyl-, Alkinyl- oder Cycloalkylgruppen gegebenenfalls durch 1 bis 3 Substituenten, die unabhängig voneinander unter Halogen, Nitro, Cyano, C₁-C₆-Alkoxy, C₁-C₆-Halogenalkoxy, C₁-C₆- Alkylthio, C₁-C₆-Halogenalkylthio, C₁-C₆-Alkyl- sulfonyl, C₁-C₆-Alkylsulfinyl, C₁-C₆-Halogen- alkylsulfonyl, C₁-C₆-Halogenalkylsulfinyl, C₃-C₆- Cycloalkyl, Phenyl und Pyridyl ausgewählt sind, substituiert sind;
R² für Cyclopropyl, das gegebenenfalls durch 1 bis 3 Halogenatome und/oder 1 bis 3 C₁-C₆-Alkyl- gruppen und/oder 1 bis 3 C₁-C₆-Halogenalkyl- gruppen substituiert ist, steht und
X¹ für Halogen, C₁-C₆-Alkoxy, C₁-C₆-Alkylsulfonat, C₁-C₆-Halogenalkylsulfonat, C₆-C₁₂-Arylsulfonat, C₁-C₆-Alkylthio, C₁-C₆-Alkylphosphat oder C₆-C₁₂- Arylphosphat steht.

20. Imidoylverbindung der Formel III nach Anspruch 19, worin R¹ für C₁-C₁₀-Alkyl steht, R² für Cyclopropyl, das durch 2 Halogenatome und/oder 1 C₁-C₆-Alkylgruppe substituiert ist, steht und X¹ für Halogen steht.

21. Imidoylverbindung der Formel III nach Anspruch 19 oder 20, worin R² für eine Cyclopropylgruppe der Formel VII gemäß obiger Definition, worin R⁶ für C₁-C₆-Alkyl steht, R⁷ und R⁸ beide für Halogen stehen und R⁹ und R¹⁰ beide für Wasserstoff stehen, steht.

22. 2,2-Dichlor-N-ethyl-1-methylcyclopropancarboximidoylchlorid der Formel III'

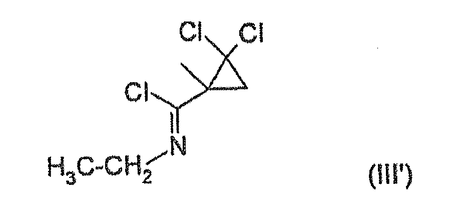

23. Amid der Formel IV 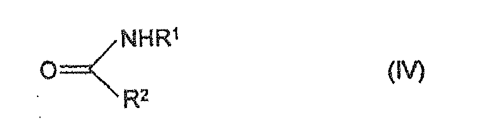 worin
R¹ für C₁-C₁₀-Alkyl, C₃-C₁₀-Alkenyl, C₃-C₁₀-Alkinyl, C₃-C₁₂-Cycloalkyl, Di(C₁-C₆-alkyl) amino, C₁-C₆- Alkylsulfonyl oder C₁-C₆-Alkylsulfinyl steht, wobei in jedem der obigen Reste die Alkyl-, Alkenyl-, Alkinyl- oder Cycloalkylgruppen gegebenenfalls durch 1 bis 3 Substituenten, die unabhängig voneinander unter Halogen, Nitro, Cyano, C₁-C₆-Alkoxy, C₁-C₆-Halogenalkoxy, C₁-C₆- Alkylthio, C₁-C₆-Halogenalkylthio, C₁-C₆-Alkyl sulfonyl, C₁-C₆-Alkylsulfinyl, C₁-C₆-Halogen- alkylsulfonyl, C₁-C₆-Halogenalkylsulfinyl und C₃-C₆-Cycloalkyl ausgewählt sind, substituiert sind; und
R² für eine Cyclopropylgruppe der Formel VII gemäß obiger Definition, worin R⁶ für C₁-C₆-Alkyl steht, R⁷ und R⁸ beide für Chlor stehen und R⁹ und R¹⁰ beide für Wasserstoff stehen, steht.

24. Amid der Formel IV nach Anspruch 23, worin R¹ für C₁-C₄-Alkyl steht.

25. 2,2-Dichlor-1-methylcyclopropancarbonsäureethylamid der Formel IV'

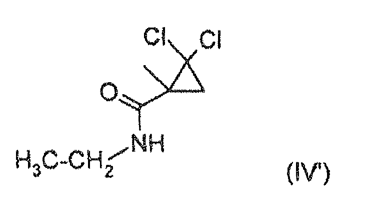

## Revendications

1. Procédé pour la préparation d'amidrazones de formule I ou d'un sel de celles-ci 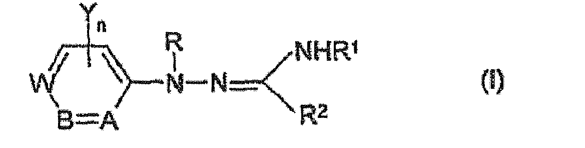 où
R représente hydrogène ;
R¹ représente C₁-C₁₀-alkyle ;
R² représente C₃-C₁₀-cycloalkyle qui est non substitué ou substitué par 1 à 5 atomes d'halogène et/ou 1 à 3 groupes C₁-C₆-alkyle et/ou 1 à 3 groupes C₁-C₆- halogénoalkyle ;
A représente C-R³ ou N ;
B représente C-R⁴ ou N ;
W représente C-R⁵ ou N ;
à condition qu'un parmi A, B et W soit autre que N ;
R³, R⁴, R⁵ représentent chacun indépendamment hydrogène, halogène, nitro, cyano, amino, mercapto, hydroxy, C₁-C₁₀-alkyle, C₂-C₁₀-alcényle, C₂-C₁₀-alcynyle, C₃- C₆-cycloalkyle, C₁-C₆-alcoxy, C₁-C₆-alkylamino, di(C₁-C₆-alkyl)-amino, C₁-C₆-alkylthio, C₁-C₆- alkylsulfonyle, C₁-C₆-alkylsulfinyle ou un cycle aromatique à 5 ou 6 chaînons qui peut contenir 1 à 4 hétéroatomes choisis parmi oxygène, soufre et azote ;
Y représente hydrogène, halogène, cyano, nitro, amino, hydroxy, mercapto, C₁-C₆-alkyle, C₂-C₁₀- alcényle, C₂-C₁₀-alcynyle, C₃-C₆-cycloalkyle, C₁-C₆- alcoxy, C₁-C₆-alkylamino, di(C₁-C₆)-alkylamino, C₁- C₆-alkylthio, C₁-C₆-alkylsulfonyle, ou C₁-C₆- alkylsulfinyle ; et
n vaut 0, 1 ou 2,
comprenant la réaction d'une arylhydrazine de formule II 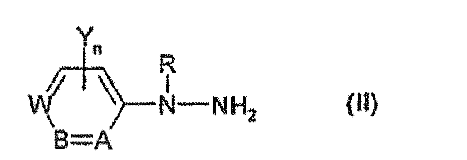 où R, A, B, W, Y et n sont chacun tel que défini ci-dessus,
avec un composé imidoyle de formule III : 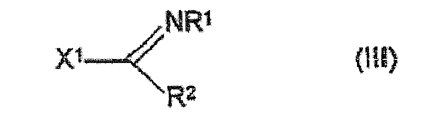 où R¹ et R² sont chacun tel que défini ci-dessus et X¹ représente halogène, C₁-C₆-alcoxy, C₁-C₆-alkylsulfonate, C₁-C₆-halogénoalkylsulfonate, C₆-C₁₂-arylsulfonate, C₁-C₆-alkylthio, C₁-C₆-alkylphosphate, ou C₆-C₁₂-arylphosphate, éventuellement en présence d'une base.

2. Procédé selon la revendication 1, dans lequel la réaction de l'arylhydrazine de formule II avec le composé imidoyle de formule III est réalisée en présence d'une base pour obtenir l'amidrazone de formule I.

3. Procédé selon la revendication 2, dans lequel la base est une amine tertiaire.

4. Procédé selon la revendication 3, dans lequel l'amine tertiaire est la tri-n-butylamine.

5. Procédé selon l'une quelconque des revendications 2 à 4, dans lequel on fait ensuite réagir l'amidrazone obtenue de formule I avec un acide
approprié pour former un sel d'addition d'acide de ladite amidrazone.

6. Procédé selon l'une quelconque de revendications 1 à 5, dans lequel le composé imidoyle de formule III est obtenu en faisant réagir un amide de formule IV 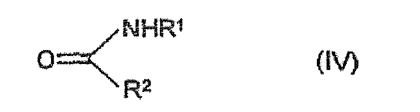 où R¹ et R² sont chacun tel que défini ci-dessus,
avec un agent apte à convertir l'amide IV en composé imidoyle III.

7. Procédé selon la revendication 6, dans lequel l'agent apte à convertir l'amide IV en composé imidoyle III est choisi parmi des agents d'halogénation, des agents d'O-alkylation, des agents de phosphatation, et des agents de sulfonylation.

8. Procédé selon la revendication 6 ou 7, dans lequel l'amide de formule IV est obtenu en faisant réagir un composé de formule V 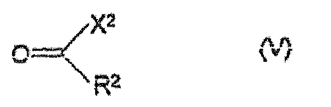 où X² représente un groupe partant approprié et R² est tel que défini ci-dessus,
avec une amine de formule VI
R¹-NH₂ (VI)
où R¹ est tel que défini ci-dessus.

9. Procédé selon l'une quelconque des revendications 1 à 8, comprenant les étapes de
(a) réaction du composé de formule V avec l'amine de formule VI pour obtenir l'amide de formule IV,
(b) réaction de l'amide de formule IV avec l'agent apte à convertir l'amide de formule IV en composé imidoyle de formule III pour obtenir ledit composé imidoyle, et
(c) réaction du composé imidoyle de formule III avec une arylhydrazine de formule II, éventuellement en présence d'une base pour obtenir l'amidrazone de formule I ou un sel de celle-ci.

10. Procédé selon la revendication 9, dans lequel les étapes (a), (b) et (c) sont réalisées dans un procédé dans un pot sans isolement de l'amide de formule IV et du composé imidoyle de formule III.

11. Procédé selon la revendication 9 ou 10, dans lequel chacune des étapes (a), (b) et (c) est effectuée dans le même solvant.

12. Procédé selon la revendication 11, dans lequel le solvant est un hydrocarbure aromatique.

13. Procédé selon l'une quelconque des revendications 1 à 12, dans lequel R² dans les formules I, III, IV et V représente cyclopropyle qui est substitué par 2 atomes d'halogène et/ou 1 groupe C₁-C₆-alkyle.

14. Procédé selon l'une quelconque des revendications 1 à 13, dans lequel R² dans les formules I, III, IV et V représente un groupe cyclopropyle de formule VII 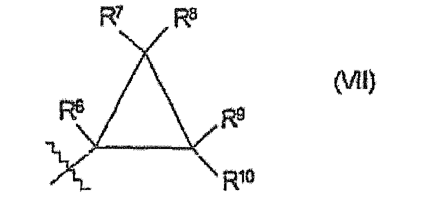 où R⁶ représente hydrogène, C₁-C₆-alkyle ou C₁-C₆-halogénoalkyle, et R⁷, R⁸ R⁹ et R¹⁰ sont choisis chacun indépendamment parmi hydrogène, halogène, C₁-C₆-alkyle ou C₁-C₆-halogénoalkyle.

15. Procédé selon la revendication 14, dans lequel R⁶ représente C₁-C₆-alkyle, R⁷ et R⁸ représentent tous les deux halogène, et R⁹ et R¹⁰ représentent tous les deux hydrogène.

16. Procédé selon l'une quelconque des revendications 1 à 15, dans lequel R² dans les formules I, III, IV et V représente 1-(2,2-dichloro-1-mêthylcyclopropyle).

17. Procédé selon l'une quelconque des revendications 1 à 16, dans lequel A dans les formules I et II désigne C-R³ où R³ représente halogène ou C₁-C₆-halogénoalkyle, B dans les formules I et II désigne C-R⁴ où R⁴ représente hydrogène ou halogène, W dans les formules I et II désigne C-R⁵ où R⁵ représente halogène ou C₁-C₆-halogénoalkyle, Y dans les formules I et II représente un atome d'halogène dans la position 6 du cycle aromatique, n dans les formules I et II vaut 1, R¹ dans les formules I, III, IV et VI représente C₁-C₄-alkyle, X¹ dans la formule III représente halogène, et X² dans la formule V représente halogène ou C₁-C₆-alcoxy.

18. Procédé selon l'une quelconque des revendications 1 à 17, dans lequel A dans les formules I et II représente C-Cl, B dans les formules I et II représente C-H, W dans les formules I et II représente C-CF₃, Y dans les formules I et II représente un atome de chlore dans la position 6 du cycle aromatique, n dans les formules I et II vaut 1, R¹ dans les formules I, III, IV et VI représente éthyle, X¹ dans la formule III représente chlore, et X² dans la formule V représente chlore ou méthoxy.

19. Composé imidoyle de formule III 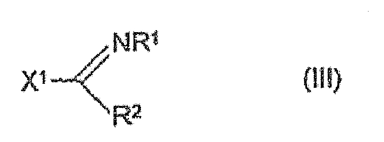 où
R¹ représente C₁-C₁₀-alkyle, C₃-C₁₀-alcényle, C₃-C₁₀- alcynyle, C₃-C₁₂-cycloalkyle, di-(C₁-C₆-alkyl)- amino, C₁-C₆-alkylsulfonyle ou C₁-C₆-alkylsulfinyle, où dans chacun des radicaux ci-dessus les groupes alkyle, alcényle, alcynyle ou cycloalkyle sont non substitués ou substitués par 1 à 3 substituants choisis indépendamment parmi halogène, nitro, cyano, C₁-C₆-alcoxy, C₁-C₆-halogénoalcoxy, C₁-C₆- alkylthio, C₁-C₆-halogénoalkylthio, C₁-C₆- alkylsulfonyle, C₁-C₆-alkylsulfinyle, C₁-C₆- halogénoalkylsulfonyle, C₁-C₆- halogénoalkylsulfinyle, C₃-C₆-cycloalkyle, phényle et pyridyle ;
R² représente cyclopropyle qui est substitué par 1 à 3 atomes d'halogène et/ou 1 à 3 groupes C₁-C₆- alkyle et/ou 1 à 3 groupes C₁-C₆-halogénoalkyle ; et
X¹ représente halogène, C₁-C₆-alcoxy, C₁-C₆- alkylsulfonate, C₁-C₆-halogénoalkylsulfonate, C₆- C₁₂-arylsulfonate, C₁-C₆-alkylthio, C₁-C₆- alkylphosphate, ou C₆-C₁₂-arylphosphate.

20. Composé imidoyle de formule III selon la revendication 19, dans lequel R¹ représente C₁-C₁₀-alkyle, R² représente cyclopropyle qui est substitué par 2 atomes d'halogène et/ou 1 groupe C₁-C₆-alkyle et X¹ représente halogène.

21. Composé imidoyle de formule III selon la revendication 19 ou 20, dans lequel R² représente un groupe cyclopropyle de formule VII tel que défini ci-dessus où R⁶ représente C₁-C₆-alkyle, R⁷ et R⁸ représentent tous les deux halogène, et R⁹ et R¹⁰ représentent tous les deux hydrogène.

22. Chlorure de 2,2-dichloro-N-éthyl-1-méthylcyclopropanecarboximidoyle de formule III'

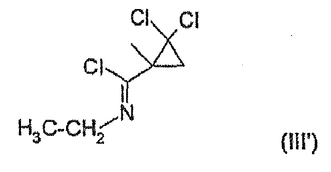

23. Amide de formule IV 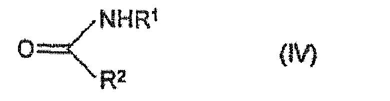 où
R¹ représente C₁-C₁₀-alkyle, C₃-C₁₀-alcényle, C₃-C₁₀- alcynyle, C₃-C₁₂-cycloalkyle, di-(C₁-C₆-alkyl)- amino, C₁-C₆-alkylsulfonyle ou C₁-C₆-alkylsulfinyle, où dans chacun des radicaux ci-dessus les groupes alkyle, alcényle, alcynyle ou cycloalkyle sont non substitués ou substitués par 1 à 3 substituants choisis indépendamment parmi halogène, nitro, cyano, C₁-C₆-alcoxy, C₁-C₆-halogénoalcoxy, C₁-C₆- alkylthio, C₁-C₆-halogénoalkylthio, C₁-C₆- alkylsulfonyle, C₁-C₆-alkylsulfinyle, C₁-C₆- halogénoalkylsulfonyle, C₁-C₆- halogénoalkylsulfinyle, C₃-C₆-cycloalkyle ; et
R² représente un groupe cyclopropyle de formule VII tel que défini ci-dessus, où R⁶ représente C₁-C₆- alkyle, R⁷ et R⁸ représentent tous les deux chlore, et R⁹ et R¹⁰ représentent tous les deux hydrogène.

24. Amide de formule IV selon la revendication 23, dans laquelle R¹ représente C₁-C₄-alkyle .

25. Éthylamide de l'acide 2,2-dichloro-1-méthylcyclopropanecarboxylique de formule IV'
